# EUROPÄISCHE PATENTSCHRIFT

(11) **EP 2 335 609 B1**
(45) Veröffentlichungstag und Bekanntmachung des Hinweises auf die Patenterteilung: **18.04.2012**
(21) Anmeldenummer: 09015641.5
(22) Anmeldetag: 17.12.2009
(51) Int. Cl.: A61B 17/122, A61B 17/128, A61B 17/00

(54) **Chirurgisches Instrument**
Surgical instrument
Instrument chirurgical

(43) Veröffentlichungstag der Anmeldung: 22.06.2011
(73) Patentinhaber: CARDIOMEDICAL GmbH, 30855 Langenhagen (DE)
(72) Erfinder: Wiedenbein, Wolfgang, 30926 Seelze (DE)

(56) Entgegenhaltungen:
- WO-A2-02/38081
- DE-U1-202007 016 057
- US-A- 6 146 392
- US-A1- 2003 212 435

## Beschreibung

### Technisches Gebiet

Die vorliegende Erfindung bezieht sich allgemein auf das Gebiet der Medizin, insbesondere der Chirurgie. Im chirurgischen Bereich werden Instrumente, Vorrichtungen oder Verfahren eingesetzt, um das Innere von lebenden Organismen zu untersuchen und/oder für operative Eingriffe zu nutzen Zu den chirurgischen Instrumenten zählen alle medizinischen Instrumente, die vornehmlich in der Chirurgie Verwendung finden. Dazu gehören auch chirurgische Instrumente zum Abbinden oder anderweitigem Zusammendrücken von röhren-, bzw. schlauchförmigen Körperteilen, vorzugsweise von Blutgefäßen. Solche chirurgischen Instrumente werden dem Bereich der fassenden, bzw. klemmenden Instrumente zugeordnet und sind in einer großen Typenvielfalt vorhanden und hinreichend bekannt. Fassende, bzw. klemmende chirurgische Instrumente werden beispielsweise bei Eingriffen in der Herz-, Thorax- und Gefäßchirurgie verwendet. In der Herz-und Thoraxchirurgie wird meistens eine offene Operation durchgeführt, bei der, durch die Eröffnung des Thorax, ein Zugang zum Herzen geschaffen wird. Der Zugang erfolgt in der Regel mittels einer medianen Stemotomie, wobei ein in etwa 25 cm langer Längsschnitt durch das Brustbein erzeugt wird. Der Längsschnitt wird zum Öffnen des Brustkorbes benötigt. Bei der Thorakotomie erfolgt die chirurgische Öffnung des Thorax durch einen Interkostalschnitt, d.h., einen kleinen Schnitt in den Rippenzwischenraum. Die durch die Stemotomie oder Thorakotomie erzeugte Öffnung wird, mittels eines Rippenspreizers, der zum Aufdehnen und Offenhalten des Brustkorbes eingesetzt wird, frei gehalten. Die Öffnung dient den Chirurgen als Zugang für operative Eingriffe. Die Eingriffe an organischen Körperteilen erfolgen dann durch die erzeugte Öffnung im Brustkorb, mit Hilfe einer Vielzahl unterschiedlicher chirurgischer Instrumente. Ist beispielsweise das Herz des Patienten freigelegt, werden direkt an das Herz und die großen Blutgefäße verschiedene Katheder, Kanülen und Klemmen angelegt. Typischerweise wird die Aorta mit einer Gefäßklemme, rund um die aufsteigende Aorta, okkludiert, um die Koronararterien vom Rest des Arteriensystems zu isolieren. Die notwendigerweise eingesetzten chirurgischen Instrumente verkleinern einerseits die Öffnung und behindern die Tätigkeit des Chirurgen in seinem Gesichtsfeld und andererseits ist ein schneller Heilungsprozess, aufgrund der Größe der Öffnung, des entstandenen Gewebeschadens und des operativen Traumas, bei dem Patienten nicht zu erwarten.

### Hintergrund im Stand der Technik

Die vorliegende Erfindung betrifft ein solches medizinisches Instrument, insbesondere ein chirurgisches Instrument, beispielsweise eine Gefäßklemme, zum Okkludieren von schlauchförmigen organischen, menschlichen oder tierischen Körperteilen, wie Blutgefäße, vorzugsweise Arterien, welches aus zwei, beweglich miteinander scherenartig verbundenen Schenkeln besteht, die am distalen Ende ein Arbeitsmittel, vorzugsweise eine Klemm- und Greifeinrichtung, die zwei Greifelemente umfasst und am proximalen Ende aus einer Betätigungseinrichtung besteht, vorzugsweise einer Griffeinrichtung, die zwei ringförmige Griffelemente umfasst, sowie nahe der ringförmigen Griffelemente aus einer Feststellvorrichtung besteht, die vorzugsweise zwei, mit Rastzähnen aufweisende Klinken am Schenkel umfasst, mit der die Greifelemente in einer von mehreren Stellungen eingestellt werden können.

Medizinische Instrumente dieser Art, insbesondere fassende und klemmende chirurgische Instrumente in verschiedenen Bauarten und Ausführungen, haben sich als chirurgische Instrumente bei operativen Eingriffen vielfach bewährt und sind aus dem Stand der Technik bekannt.

Ein bekanntes Beispiel ist die Kocherklemme. Die Kocherklemme ist eine traumatische Klemme und gehört in die Klasse der zufassenden chirurgischen Instrumente. Eine solche Klemme wird vorwiegend dann eingesetzt, wenn Strukturen vornehmlich sicher gefasst, gehalten und dabei komprimiert werden müssen. Zu diesem Zweck hat die Kocherklemme geriffelte Branchen, damit das gefasste Gewebe nicht antegrad aus den Branchen rutscht.

Bekannt ist auch die gebogene Aortenklemme nach Huland und Noldus, welche, um Rektrumverletzungen zu vermeiden, zur Präparation zwischen Rektrum und Denonvillierscher Faszie bei der Prostatektomie eingesetzt wird.

Die dabei verwendete Verzahnung der Branchen ist bereits von dem Erfinder Elvin E.Baker in der US 2,668,538 offenbart worden. Diese Instrumente besitzen aber den Nachteil, dass eine Beschädigung des Gewebes nicht ausgeschlossen ist.

Eine solche, aus der großen Typenvielfalt hergestellte chirurgische Klemme, ist der DE 27 47 625 A1 zu entnehmen. Bei der Klemme wurde speziell der Handgriff verändert. Aus der DE 25 21 4878 C2 ist ersichtlich, dass die Klauen eine gebogene Form aufweisen. Stellvertretend für die vielen verschiedenartigen Klemmen sei hier die chirurgische Klemme aus der DE 2 060 814 A genannt. Die offenbarte Klemme ermöglicht, ohne die Anwendung scharfer Kanten in der Verzahnung, und somit scharfer Kanten am Gewebe, wodurch ungewollte Verletzungen des empfindlichen Gewebes verhindert werden, einen sicheren Klemmvorgang. Jede Branche weist wenigstens eine Längsrippe mit einer Reihe von Zähnen und mindestens eine Längsnut, zur Aufnahme der Zähne der Längsrippe der gegenüberliegenden Branche, auf. Die Zähne der Rippen auf der zugehörigen Branche sind seitlich versetzt und relativ zueinander angeordnet, und die Rippen auf den zugehörigen Branchen besitzen längs verlaufende Flanken, die in der Klemmstellung der Branchen nahe beieinander liegen und so geformt sind, dass sie eine längsverlaufende, gewebeaufnehmende Tasche, mit im Querschnitt vergrößertem Mittelabschnitt und verkleinerten Endabschnitten, bilden.

Der Abklemmvorgang von Blutgefäßen durch die zuvor aufgezählten chirurgischen Instrumente erfolgt nach dem gleichen Prinzip. Die beiden Branchen der chirurgischen Instrumente werden mit Hilfe von Ringgriffen aufeinander zu und voneinander weg bewegt. Eine Klink- oder Sperreinrichtung, mit der die Branchen in einer von mehreren einzelnen Stellungen eingestellt werden können, ist an den Scherengliedern angeordnet. An den beiden Scherengliedern befinden sich jeweils eine Klinke und mit dieser zusammenwirkende Zähne, bzw. sind an den Scherengliedern Zahnleisten mit Rastzähnen als Arretierungseinrichtung angeordnet. Die Einstellung der Abstände der Branchen zueinander kann also nur in Stufen erfolgen. Der große Nachteil bei einer Stufeneinteilung ist, dass ein Gefäß entweder zu stark oder zu schwach abgeklemmt wird.

Eine weitere Ausführungsform einer Aortenklemme kann dem Produktkatalog "HORIZON" der Firma Cardiomedical GmbH, auf der Homepage unter Cardio Vision auf der Seite MIC-Accessoires, nebst Abbildung, entnommen werden. Die im Produktkatalog aufgezeigte Aortenklemme ist im Prinzip auch aus der DE 696 32 451 T2 der Fig.4, ersichtlich.

Um die Nachteile der scherenartigen, mit ringförmigen Griffen ausgebildeten Klemmen und der, Rastzähne aufweisende Klinken die eine Sperrvorrichtung bilden, zu vermeiden, schlägt die DE 20 2007 016 057 U1 eine stufenlose Verstellung der Greifelemente für eine laparoskopische Aortenklemme vor. Aortenklemmen mit einer stufenlosen Verstellung der Greifelemente, bzw. der Branchen, sind auch der Homepage www.eisold-instruments.com, unter der Rubrik Produkte "Aortenklemmen", zu ersehen.

Als nächstliegender Stand der Technik wird der Gegenstand aus der US 2003/0212435 A1 angesehen. Der offenbarte Gegenstand betrifft beispielweise, nach Absatz 2, 30 und der Figur 6 im veröffentlichten Dokument, ein zerleg- und komplettierbares chirurgisches Instrument, welches aus einer Bedieneinrichtung, einer stufenlos fassenden und klemmenden Gefäßklemme, die ein Körperelement, ein Arbeitsmittel, eine Verstelleinrichtung, eine Koppeleinrichtung umfasst und einem Betätigungswerkzeug, welches fest mit der Bedieneinrichtung verbunden ist, gebildet wird.

Eine Gemeinsamkeit aller vorgenannten chirurgischen Instrumente ist deren Größe, die scherenartige Form mit überwiegend ringförmigen Griffen zur Betätigung der Branchen, und eine typische Sperreinrichtung.

Aus dem Bereich der chirurgischen Instrumente sind aus dem Stand der Technik auch Nadelhalter bekannt. Diese Nadelhalter sind ähnlich wie die zuvor aufgezeigten chirurgischen Gefäßklemmen aufgebaut. Diese Nadelhalter-Instrumente weisen idR. ebenfalls zwei beweglich miteinander scherenartig verbundene Schenkel auf. Die Schenkel sind an ihrem distalen Ende mit einer Greifvorrichtung, bestehend aus zwei Greifklauen und an ihrem proximalen Ende mit zwei ringförmigen Griffen ausgebildet. Ein solcher chirurgischer Nadelhalter wird beispielsweise in der US 6,146.392 offenbart. Gemäß der Beschreibung und den Figuren ist ersichtlich, dass das Instrument aus einer Bedieneinrichtung und einem Nadelhalter gebildet wird. Die Bedieneinrichtung weist an ihrem proximalen Ende Griffelemente und mit stufigen Rastzähnen, sogenannter Rastverschluss, ausgebildete Klinken auf. Am distalen Ende der Bedieneinrichtung ist eine Greifeinrichtung angeordnet. Die Greifeinrichtung besteht aus zwei Klauen zum Halten eines Nadelhalters. Um den Nadelhalter in die Greifeinrichtung der Bedieneinrichtung einzuführen, werden die Arme des Nadelhalters vom Benutzer zusammengedrückt und der Nadelhalter zwischen die Klauen eingeführt. Der zwischen den Klauen der Greifeinrichtung eingespannte Nadelhalter ist über eine Verbindungsstange mit einem zusätzlichen ringförmigen Griff verbunden. Der ringförmige Griff ist an einem Schenkel eines Griffelementes angeordnet. Mit Hilfe des ringförmigen Griffes und der Verbindungsstange kann der, in der Greifeinrichtung angeordnete Nadelhalter, gedreht werden, wobei der Nadelhalter aus zwei Armen besteht. Jeder Arm weist mehrere Bohrungen auf. In eine Bohrung eines jeden Armes greift ein, an einer Klaue der Greifeinrichtung angeordneter und nach innen weisender Zapfen, ein. Um diesen Zapfen ist der Nadelhalter drehbar angeordnet. Zur Ausführung einer Drehung des Nadelhalters greift in eine weitere Bohrung eines jeden Armes die Verbindungsstange ein. Bei der Betätigung des ringförmigen Griffes wird die Verbindungsstange verschoben und somit der Nadelhalter gedreht. Des Weiteren sind an den Innenseiten der Arme des Nadelhalters konvexe Nadelpositionierungsnuten zur Aufnahme einer Nadel angeordnet. Ist eine Nadel zwischen den Armen geklemmt, kann die Nadel entlang einer kreisförmigen Bahn bewegt werden.

Jüngste Entwicklungen in der Herz-, Thorax- und Gefäßchirurgie, welche einen Weg zur minimalinvasiven Chirurgie aufzeigen, reduzieren die Größe der Zugangsöffnung im Thorax, vor allem bei der Thorakotomie, um den Gewebeschaden und das operative Trauma des Patienten zu vermindern und eine Beschleunigung des Heilungsprozesses zu erzielen. Kleine Zugangsöffnungen haben für den Chirurgen den Nachteil, dass die, in der Öffnung notwendigerweise eingesetzten chirurgischen Instrumente, das Operationsfeld, bzw. das Gesichtsfeld wesentlich verkleinern, wodurch die Operationstätigkeit erschwert wird.

Der Weg in die minimalinvasive Chirurgie kann daher nur erfüllt werden, wenn die verwendeten chirurgischen Instrumente den neuen Vorgaben, der kleineren Öffnungen, angepasst werden. D.h., die Ausführungsformen der chirurgischen Klemmen entsprechen nicht

mehr den medizinischen Anforderungen bei den operativen Eingriffen. Operative Eingriffe werden, um die Belastungen des Patienten und das Risiko von Nebenwirkungen zu verringern, auch zeitlich schneller durchgerührt. Dazu werden atraumatische Instrumente benötigt deren Handhabung (handling) den operativen Eingriff wesentlich erleichtert.

Diese Aufgaben werden zum Teil von dem aus der US 2003/0212435 A1 offenbarten Gegenstand erfüllt. Der Gegenstand betrifft eine zerleg- und komplettierbare chirurgische Klemme. Aufgrund der Zerlegbarkeit kann das Erfordernis von kleineren Instrumenten bei der minimalinvasiven Chirurgie erfüllt werden. Der Nachteil dieser zerlegbaren chirurgischen Klemme besteht darin, dass das Kriterium des optimalen Handlings bei der Operation aber nicht erfüllt ist.

Der Erfindung liegt daher die Aufgabe zugrunde, ein medizinisches Instrument der eingangs genannten Art, für den Einsatz in der Chirurgie, zu schaffen, welches die vorgenannten Nachteile und Unzulänglichkeiten der bekannten Anordnungen vermeidet und eine technische Lösung anzugeben, die es ermöglicht, ein, mit einfacher Funktionsgeometrie ausgestattetes chirurgisches Instrument für die erhöhten Ansprüche zu schaffen. Das chirurgische Instrument soll die gleichen Eigenschaften wie die, aus dem Stand bekannten chirurgischen Klemmen, und ein vorteilhaftes Handling aufweisen und trotzdem das Operationsfeld, bzw. das Gesichtsfeld des Operateurs, nicht verkleinern, sondern vergrößern.

Erfindungsgemäß werden diese Probleme durch die Merkmale des Patentanspruchs 1 gelöst. Vorteilhafte Ausgestaltungen und Weiterbildungen der Erfindung ergeben sich aus den nachfolgenden Unteransprüchen und den nachstehenden Beschreibungen.

### Beschreibung der Erfindung

Um ein mit diesen Merkmalen der vorliegenden Erfindung ausgestattetes medizinisches Instrument, insbesondere chirurgisches Instrument, zur Verwendung bei operativen Eingriffen am menschlichen oder tierischen Körper, in der Chirurgie herzustellen, wird erfindungsgemäß vorgeschlagen, das chirurgische Instrument so zu gestalten, dass dem Chirurgen eine ergonomisch gestaltete Bedieneinrichtung zur Verfügung steht, welche die Handhabung des chirurgischen Instruments, insbesondere einer Gefäßklemme, wesentlich erleichtert. Um ein derartig ausgebildetes chirurgisches Instrument zu erhalten, mussten die zuvor genannten Aufgaben gelöst werden. Die Lösung der Aufgaben bestehen darin, die bisher bekannten scherenartigen, mit ringförmigen Griffen und stufenförmigen Sperrmechanismus ausgebildeten chirurgischen Instrumente, durch ein erfindungsgemäßes chirurgisches Instrument zu ersetzen.

Hierzu wurde vorgeschlagen, eine Kombination aus den bekannten scherenartigen Klemmen und den Klemmen mit stufenloser Verstellung der Greifelemente, wie auch aus der US 2003/0212435 A1 bekannt, vorzunehmen.

Das aus der Entwicklung entstandene chirurgische Instrument wurde erfindungsgemäß, um die Öffnung im Thorax von dem, bei der Operation benötigten chirurgischen Instrument frei zu halten, dreiteilig zerleg- und komplettierbar ausgeführt. Die drei zerleg- und komplettierbaren Bauteile des erfinderischen chirurgischen Instruments betreffen vorteilhaft eine Bedieneinrichtung, eine Gefäßklemme und ein Betätigungswerkzeug. D.h., die Lösung der drei Aufgaben das Handling einer zerleg- und komplettierbaren chirurgischen Klemme vorteilhaft zu verbessern, findet sich in den erfinderischen Merkmalen, die in den Ansprüchen aufgeführt sind, wieder. Die vorteilhaften Ausführungen des erfinderischen chirurgischen Instruments können der Beschreibung entnommen werden.

Das zerleg- und komplettierbare medizinische Instrument ist erfindungsgemäß dreiteilig in eine Bedieneinrichtung, eine Gefäßklemme und ein Betätigungswerkzeug aufgeteilt. Die am distalen Ende der Bedieneinrichtung an der Greifeinrichtung ausgebildeten Greifelemente weisen eine u-förmige Ausgestaltung auf und sind mit einem Betrag, der sich aus der Breite der Koppeleinrichtung der Gefäßklemme ergibt, parallel beabstandet. Die innen liegenden Arbeitsflächen der Greifelemente weisen eine strukturierte Oberfläche auf und ein Schenkel und eine Klinke der Bedieneinrichtung sind Träger eines daran angeordneten Führungselementes, welches aus einem Hohlzylinder gebildet wird und der eine Länge aufweist, die in etwa der Gesamtlänge eines Schenkels einschließlich Griffelement entspricht. Der Hohlzylinder besteht aus einem metallischen Rohr, welches mit Hilfe eines thermischen Verfahrens an dem Schenkel befestigt ist und eine durchgehende Bohrung aufweist, die der Aufnahme, Führung und Entnahme eines Betätigungswerkzeuges dient, welches zur Betätigung einer Verstellvorrichtung eingesetzt wird.

Die Wirkung der vorteilhaften Zerleg- und Komplettierbarkeit des chirurgischen Instruments besteht also darin, dass das chirurgische Instrument vor der Operation komplettiert und während der Operation, bzw. während der Benutzung, zerlegt oder komplettiert werden kann. Komplettieren heißt, dass die Bedieneinrichtung mit ihrer, daran angeordneten Greifeinrichtung, eine Gefäßklemme erfassen und mit Hilfe der an der Betätigungseinrichtung angeordneten Feststellvorrichtung klemmen und somit festhalten kann, und dass durch das, an der Bedieneinrichtung vorteilhaft angeordnete Führungselement, bestehend aus einem Hohlzylinder, ein Betätigungswerkzeug eingeschoben werden kann, welches in die Verstelleinrichtung einer Gefäßklemme eingreifen kann.

Die Benutzung des Betätigungswerkzeuges kann vorteilhafterweise zu unterschiedlichen Zeiten erfolgen. Das Betätigungswerkzeug kann beispielsweise direkt nach dem Erfassen einer Gefäßklemme zum Einstellen der Branchen, welche das Arbeitsmittel der Gefäßklemme darstellen, durch das Führungselement eingeschoben werden. In einem anderen Beispiel kann das Betätigungswerkzeug aber auch erst nach dem Einführen der chirurgischen Klemme durch eine Öffnung im Körper, benutzt werden.

Drehungen am Handgriff des Betätigungswerkzeuges erlauben dann ein dosiertes, stufenloses Schließen oder Öffnen der Branchen der Gefäßklemme. Um Drehungen am Betätigungswerkzeug zum Öffnen oder Schließen der Branchen an einer Gefäßklemme in der gewünschten Richtung vornehmen zu können, weist der Handgriff Drehrichtungsmarkierungen auf. Eine Drehrichtungsmarkierung besteht aus einer Beschriftung und einem Bildelement, welches einen Pfeil darstellt. Für die Drehrichtung der Branchen, beispielsweise beim "Öffnen", lautet die Beschriftung "OPEN". Neben dem Schriftzug ist das Bildelement mit dem Pfeil nach rechts angeordnet. Das bedeutet, dass sich beim Drehen des Betätigungswerkzeuges im Uhrzeigersinn die Branchen der Gefäßklemme öffnen. Zum Schließen der Branchen erfolgt eine Drehung des Betätigungswerkzeuges entsprechend der Beschriftung "CLOSE" mit einem Pfeil nach links weisend, also entgegen dem Uhrzeigersinn.

Es besteht also für den Chirurgen auch die Möglichkeit, das chirurgische Instrument, ohne das Betätigungswerkzeug, durch die Öffnung des Thorax einzuführen und erst nachträglich das Betätigungswerkzeug durch das Führungselement, welches an der Bedieneinrichtung angeordnet ist, zu schieben, bis dieses in die Verstelleinrichtung der Gefäßklemme eingreift. Im nächsten Schritt erfolgen einige Drehungen am Handgriff des Betätigungswerkzeuges, zum Öffnen der, an der Gefäßklemme angeordneten Branchen, um ein Gefäß zu umfassen, beispielsweise eine Aorta. Entgegengesetztes Drehen am Handgriff des Betätigungswerkzeuges lassen die Branchen sich stufenlos und dosiert schließen. Beim Schließen wird ein sicheres Okkludieren, auch sklerotischer Gefäße, gewährleistet. Ist ein Gefäß okkludiert, kann das Betätigungswerkzeug aus der Bedieneinrichtung entfernt, bzw. herausgezogen werden. Im nächsten Schritt wird die Feststelleinrichtung, durch Entriegeln der Rastzähne an den Klinken gelöst, wodurch die Bedieneinrichtung aus der Öffnung des Thoraxs entfernt werden kann, um den Chirurgen die größtmöglichste Öffnung für die Operation zu bieten.

Ein Betätigungswerkzeug besteht daher aus einer zylindrischen Führungstange, die um einiges länger ist als die Länge des Hohlzylinders und die der korrespondierenden Bohrung des Hohlzylinders spielfrei angepasst ist. An der zylindrischen Führungsstange ist an einem Ende ein Handgriff mit einer Drehrichtungsmarkierung und am anderen Ende eine Klinge angeordnet, wobei die Größe der Klinge kleiner als die Bohrung im Hohlzylinder ist.

Bei der Anwendung der erfindungsgemäßen chirurgischen Instrumente, welche in offenen chirurgischen Prozeduren und/oder in laparoskopischen chirurgischen Prozeduren eingesetzt werden, wird mit Hilfe der, an dem chirurgischen Instrument angeordneten Gefäßklemme, bestehend aus einem Arbeitsmittel und einer Verstelleinrichtung, eine Gefäßabklemmung ermöglicht. Das Abklemmen erfolgt über das Arbeitsmittel, welches dazu zwei Branchen aufweist, die speziell zum Abklemmen schlauchförmiger Gefäße des Körpers ausgelegt sind, und eine nekrotische Zerstörung von Gewebe ausschließen. Die Vermeidung einer traumatischen Beschädigung und ein sicherer Verschluss eines Gefäßes, oder eines ähnlich festzuhaltenden Gewebes, wird durch den Einsatz verschiedenartig genuteter, gezahnter und perforierter Oberflächen der Branchen, gemäß der Offenlegungsschrift DE 2060814, erreicht. Auch können die Branchen einer Gefäßklemme in Längsrichtung der Backe mit einer Nut versehen sein. Diese Anordnung einer Nut in den Backen hat den Vorteil, dass ein auswechselbarer Polstereinsatz aus beispielsweise thermoplastischen Elastomer in diese Nut eingesetzt werden kann. Ein solcher auswechselbarer Polstereinsatz ist beispielsweise aus der US 6,692,514 B2, der US 6,719,766 B1 und der US 2005/0059987 A1 ersichtlich.

Durch die anschließende Betätigung der Betätigungseinrichtung wird die Feststellvorrichtung an der Bedieneinrichtung gelöst, wodurch die Greifelemente der Greifeinrichtung geöffnet werden. Das Öffnen der Greifeinrichtung löst die Verbindung zwischen der Gefäßklemme und der Bedieneinrichtung und die Bedieneinrichtung kann ebenfalls aus der Öffnung im Thorax entfernt werden. Die Gefäßklemme verbleibt am okkludierten Körperteil, bzw. Gefäß. Eine vorteilhafte Zerlegung des chirurgischen Instruments in seine drei Bauteile ist somit erfolgt. Die Thoraxöffnung ist damit frei von störenden chirurgischen Instrumententeilen. Je mehr Gefäßklemmen von ihrer Bedieneinrichtung, bzw. störenden Instrumententeilen, getrennt werden können, desto größer verbleibt die, für die Operation benötigte, Öffnung im Thorax, bzw. desto kleiner kann die Öffnung des Zuganges vorteilhafterweise ausfallen. Zum Ende der durchgeführten operativen Maßnahmen werden die, zur Operation benötigten und eingesetzten unterschiedlichen chirurgischen Instrumente, wieder aus der Körperhöhle entnommen. D.h., die angelegten Katheder, Kanülen und Klemmen werden durch die Zugangsöffnung im Thorax entfernt. Zum Entfernen der erfinderischen Gefäßklemmen wird die Bedieneinrichtung wieder an eine Gefäßklemme mechanisch angekoppelt. Die Gefäßklemme weist deshalb eine Koppeleinrichtung auf, die von der, an der Bedieneinrichtung angeordneten Greifeinrichtung erfasst wird. Die Erfassung erfolgt derart, dass durch die Betätigung der Betätigungseinrichtung, bzw. durch das Aufeinanderzubewegen der Griffelemente, ein Aufeinanderzubewegen der Greifelemente der Greifeinrichtung erfolgt, bis die Greifelemente in dem dafür vorgesehenen Koppelbereich der Gefäßklemme fest zum Anliegen kommen. Die zusammenwirkenden Rastzähne an den Klinken der Feststellvorrichtung an der Bedieneinrichtung verhindern ein Lösen der Greifeinrichtung von der Gefäßklemme. Als nächstes wird das Betätigungswerkzeug mit seiner, an der zylindrischen Führungsstange angeordneten Klinge, in die Bohrung des Führungselementes eingeführt und bis zur Verstelleinrichtung geschoben, bis die Klinge, vorzugsweise bestehend aus einem Inbusprofil, in das Verstellelement der Verstelleinrichtung eingreift. Das erfinderische chirurgische Instrument ist damit wieder komplettiert.

Nach dem Eingreifen der Inbusklinge in das Inbusprofil des Verstellelementes erfolgen am Handgriff des Betätigungswerkzeuges einige Drehungen, um die Branche zu öffnen und die Gefäßklemme vom organischen Körperteil zu lösen. Aufgrund der stufenlosen Verstelleinrichtung wird ein kontrolliertes Freigeben eines okkludierten Gefäßes ermöglicht. Dem Öffnen und Entfernen der Gefäßklemme, insbesondere der Aortenklemme, kommt daher besondere Bedeutung zu. Das Verstellelement der Verstelleinrichtung, welches wie ein Drehverschluss wirkt, ist mit Feingewinde ausgebildet und erlaubt ein dosiertes Öffnen der Gefäßklemme, um z.B. ein Ausschlotten einer Gefäßprothese zu ermöglichen. Nach dem weiteren Öffnen der Branchen wird das komplette chirurgische Instrument entfernt.

Das erfinderische, zerleg- und komplettierbare chirurgische Instrument, ist derart ausgebildet, dass verschieden ausgestaltete Gefäßklemmen, bzw. deren verschiedenartig ausgeführte Branchen, mit ein und der gleichen Bedieneinrichtung, sowie dem gleichem Betätigungswerkzeug, kombiniert werden können. Die unterschiedlichen Gefäßklemmen weisen jeweils einen identischen Koppelbereich auf, der mit ein und der gleichen korrespondierenden Greifeinrichtung der Bedieneinrichtung erfasst werden kann. Auch das Verstellelement der Verstelleinrichtung der unterschiedlichen Gefäßklemmen ist gleich und korrespondiert mit der Klinge des Betätigungswerkzeuges. Aufgrund der Kombinationsmöglichkeiten zwischen verschiedenen Gefäßklemmen und einer Bedieneinrichtung, lassen sich vorteilhafterweise die Anzahl der Bedieneinrichtungen bei den chirurgischen Instrumenten reduzieren.

Die Lösung der ersten Aufgabe besteht darin, eine stufenlos fassende- und klemmende Gefäßklemme mit einem Körperelement zu entwickeln, wobei das Körperelement aus einem festen Körperteil I dem Körperelement I und einem beweglichen Körperteil II dem Zug-und Druckelement besteht. Das Körperelement soll an einem Ende ein Arbeitsmittel aufweisen, vorzugsweise eine Klemm- und Greifeinrichtung. Die Klemm- und Greifeinrichtung besteht aus zwei Branchen, wobei die eine Branche einstückig am distalen Ende des Körperelementes I ausgebildet und mit diesem fest verbunden ist, wobei die andere Branche über einen Stift I mit dem Zug- und Druckelement beweglich verbunden und um den Stift I drehbar, gemäß der DE 20 2007 016 057 U1, angeordnet ist. Die Branchen sind mit einer vorteilhaften Verzahnung, entsprechend der DE 2 060 814 A, ausgestattet. Des Weiteren sind am proximalen bzw. anderen Ende des Körperelementes eine Verstelleinrichtung, eine Arretierungseinrichtung und eine Koppeleinrichtung angeordnet, wobei die Verstelleinrichtung mit der Arretierungseinrichtung über das Verstellelement in Verbindung steht.

Erfindungsgemäß befindet sich die Verstelleinrichtung direkt im Anschluss an die beweglich angeordnete Branche, wodurch das Bauteil der Gefäßklemme wesentlich verkürzt wird. Die Verstelleinrichtung besteht aus einer abgewinkelten Halterung, einer Antriebswelle und einem Verstellelement, wobei dem Verstellelement eine Doppelfunktion zukommt. Das Verstellelement ist ein Teil der Verstelleinrichtung und gleichzeitig ein Teil der Arretierungseinrichtung. Zur Doppelfunktion des Verstellelementes wird nachstehend noch ausgeführt. Die abgewinkelte Halterung weist die gleiche Breite wie das feste Körperelement I auf und steht senkrecht auf der Längsachse des Körperelementes I und senkrecht zur Längsachse des Zug- und Druckelementes und ist am proximalen Ende des Körperelementes I angeordnet. Die Halterung bildet einen geometrischen Körper, vorzugsweise einen quaderförmigen Körper. Der quaderförmige Körper entspricht einem rechteckigen Stab, der im Winkel von 90 Grad zum Körperelement an diesem angeordnet ist. Die stabförmige Halterung bildet mit dem Körperelement eine L-förmige Anordnung. Die Halterung enthält eine Öffnung, vorzugsweise ausgebildet als Bohrung. Die Bohrung ist derart in der Halterung angeordnet, dass die Mittellinie der Öffnung mit der Mittellinie der Längsachse des Zug- und Druckelementes fluchtet. D.h., die Mittelinie der Bohrung verläuft parallel beabstandet zur Mittellinie des Körperelementes I und auf der gleichen Mittellinie mit der Gewindebohrung im Zug- und Druckelement.

Die Bohrung dient der Aufnahme und Führung einer Antriebswelle, die rechts und links aus der Halterung hervorsteht. Die Antriebswelle stellt im Prinzip das Verbindungselement zwischen dem Zug- und Druckelement und dem Verstellelement dar. Die Antriebswelle greift mit dem einen vorstehenden Wellenende I in das verschiebbare Zug- und Druckelement ein und nimmt mit dem anderen vorstehenden Wellenende II ein Verstellelement auf. Dabei ist die Antriebswelle zylindrisch ausgebildet und weist drei Funktionsbereiche auf Der Funktionsbereich I umfasst ein Wellenende I, welches als Gewindebolzen mit Feingewinde ausgebildet ist und das in die Gewindebohrung des verschiebbaren Zug- und Druckelementes eingreift. Während der Funktionsbereich II weitestgehend dem mittleren Teil der Antriebswelle entspricht, der aus zwei beabstandeten Flanschen, zur Aufnahme der Halterung, gebildet wird. Nach der Konfektionierung der Antriebswelle in die Bohrung der Halterung befindet sich die Halterung zwischen den beiden Flanschen der Antriebswelle, wodurch die Antriebswelle eine Führung erhält. Die Halterung und die Flansche sind dabei gemäß DIN einem Passmaß beispielsweise "H7" (aus der Tabelle Welle/Bohrung) angepasst, sodass die Antriebswelle spielfrei geführt wird. Bleibt noch der Funktionsbereich III, der dem anderen Wellenende II entspricht und aus einem zylindrischen Zapfen gebildet wird und ein Verstellelement aufnehmen kann. Der zylindrische Zapfen kann beispielsweise ebenfalls mit einem Gewinde ausgestattet sein. Das Verstellelement weist daher auf einer Seite eine Öffnung auf, die in der Größe mit dem zylindrischen Zapfen und dessen Ausführung korrespondiert. Auf der gegenüberliegenden Seite weist das Verstellelement eine Vertiefung auf, welche ein Innenprofil zur Aufnahme des Betätigungswerkzeuges enthält. Die Vertiefung ist zentrisch angeordnet und kann die Klinge eines Betätigungswerkzeuges aufnehmen. Mit dem Betätigungswerkzeug ist es jetzt möglich das Zug- und Druckelement zu verschieben.

In einer anderen Ausführung ist die Antriebswelle einstückig mit dem Verstellelement ausgebildet. In der einstückigen Ausführung entfällt das Wellenende II mit dem zylindrischen Zapfen. Zur Komplettierung des Verstellelementes mit der Halterung weist die Halterung daher einen Schlitz auf, der mit der Bohrung in Verbindung steht, durch welchen die Antriebswelle des Verstellelementes eingelegt werden kann. Zum Verschließen des Schlitzes ist ein Verschlusselement vorgesehen, welches die Antriebswelle des Verstellelementes in der Bohrung zentriert führt. Das Verstellelement ist im Prinzip dem Verbindungselement einer Schraube nachempfunden. Der Bolzen einer Schraube entspricht der zylindrischen Antriebswelle, die hier mit speziellen Flanschen ausgestattet ist, und der Schraubenkopf entspricht dem Verstellelement, welches speziell am Umfang mit einem Rastprofil für die Arretierung mit einer Rastfeder ausgestattet ist. Zentrisch weist der Schraubenkopf ein innen liegendes Formelement auf. Das Formenelement entspricht einem Innenprofil, vorzugsweise einem Innensechskantprofil zur formschlüssigen Verbindung mit einem Werkzeug, hier der Klinge des Betätigungswerkzeuges.

Das Zug- und Druckelement ist am distalen Ende drehbar mit einem Stift II verbunden, der in der beweglichen Branche angeordnet ist, und am proximalen Ende mit dem Verstellelement. Durch diese Anordnung der beiden Verbindungen an den beiden freien Enden des Zug- und Druckelementes, wird das Zug- und Druckelement parallel zum Körperelement angeordnet und gleitet auf der Oberfläche des Körperelementes. Wenn das Verstellelement gedreht wird, verschiebt sich das Zug- und Druckelement axial gegen das Körperelement, wobei der Stift II am distalen Ende des Zug- und Druckelementes ebenfalls eine Bewegung ausführt, bzw. auf einer Kreisbahn verschoben wird. Durch die Verschiebung des Zug- und Druckelementes kann die bewegliche Branche geöffnet oder geschlossen werden. Die Verschiebung erfolgt durch das Drehen am Verstellelement. Das Drehen am Verstellelement bewirkt eine stufenlose Verstellung der Branchen. Dazu weist das Verstellelement ein Innenvielkantprofil, vorzugsweise ein Innensechskantprofil, auf. Das Innensechskantprofil, auch als Inbusprofil bezeichnet, nimmt zur Verstellung der Branchen ein Betätigungswerkzeug auf. Mit dem Betätigungswerkzeug wird eine entfernte Bedienung einer erfinderischen Gefäßklemme, bzw. deren Branchen, ermöglicht. Die stufenlose Verstellung der Branchen wird über ein Gewinde, vorzugsweise Feingewinde, erreicht, welches, wie bereits zuvor erläutert, am Verstellelement angeordnet ist und das Verstellelement mit dem Zug- und Druckelement verbindet. Mit einer stufenlosen Einstellung können die Branchen in jeder beliebigen Stellung, bzw. jedem beliebigen Öffnungswinkel, ein Gewebe oder Gefäß klemmen. Die Klemmwirkung wird durch das, am Verstellelement vorhandene Gewinde, erreicht. Das Gewinde hat eine selbsthemmende Wirkung. Die stufenlose Einstellung der Branchen bietet weitere Vorteile, die der DE 20 2007 016 057 U1 entnommen werden können. Ein Vorteil ist die Einstellung der Druckkraft, die dosiert auf ein Gewebe oder Gefäß eingestellt werden kann. Daher besteht auch die Möglichkeit, bei einem Gefäß den Durchfluss einer Flüssigkeit zu regeln. Für den Operateur heißt das, so weichteilschonend wie möglich vorgehen zu können, also atraumatisch zu operieren und den Vorteil zu nutzen, der in der Zuverlässigkeit besteht, dass das Gefäß sicher mechanisch verschlossen werden kann.

Des Weiteren bildet das Verstellelement, ausgestattet am Umfang mit einem Profil, ein Arretierungsmittel. Das Profil am Umfang entspricht einem Rastprofil, vorzugsweise ausgeführt als Rillenprofil, das mit einer Rastfeder im Kontakt steht. Das Verstellelement ist somit nicht nur Teil einer Verstelleinrichtung, sondern auch Teil einer Arretierungseinrichtung. Die Arretierungseinrichtung wird somit aus einer Rastfeder und einem Verstellelement gebildet. Die Rastfeder ist aus dem Werkstoff Stahl, vorzugsweise aus Federstahl hergestellt und am Körperelement befestigt. Die Befestigungsstelle der Rastfeder befindet sich mittig an der Unterseite des Körperelementes bzw. auf der abgewandten Seite der Halterung in der Nähe des Eckbereiches am proximalen Ende des Körperelementes. Die Rastfeder ist derart gestaltet, dass diese aus zwei Schenkeln I, II gebildet wird. Die beiden Schenkel I, II sind winklig zueinander angeordnet und bilden einen Winkel von in etwa 90 Grad. Der Schenkel I bildet den horizontalen Befestigungsschenkel I und der Schenkel II bildet den vertikalen beweglichen Schenkel II. Der vertikale Schenkel II kann in zwei Richtungen ausgelenkt werden. Die Auslenkung ist relativ kurz und kann im Uhrzeigersinn oder entgegen dem Uhrzeigersinn erfolgen. Je nachdem, welche Drehrichtung am Verstellelement, mit Hilfe des Betätigungswerkzeuges, zum Öffnen und Schließen der Branchen erfolgt.

Jede Drehung am Verstellelement, ob Links- oder Rechtsdrehung, erzeugt, hervorgerufen durch die Bewegung, bzw. Auslenkung der Rastfeder, ein Geräusch. Das Geräusch ähnelt einem Klickgeräusch einer Ratsche. Das Klickgeräusch entsteht beim Wechsel der Rastfeder von einer Rille in die nächste Rille des Profils, genau genommen bei dem Übergang von einer Rille des Profils in die nächste Rille. Zusätzlich wirkt die Rastfeder im Zusammenspiel mit dem, am Umfang des Verstellelementes angeordneten Querrillen, als Arretierungsmittel. Daraus folgt, dass das Köperelement I der Gefäßklemme nicht nur Träger der Verstelleinrichtung sondern auch Träger der Arretierungseinrichtung ist.

Erfindungsgemäß befindet sich am entgegengesetzten Ende der festen Branche des Körperelementes eine Koppeleinrichtung. Die Koppeleinrichtung ist zweiteilig ausgeführt und beidseitig des Körperelementes und der Halterung der Gefäßklemme angeordnet. Die Koppeleinrichtung dient der Greifeinrichtung der Betätigungseinrichtung zum Erfassen der erfinderischen Gefäßklemme. Die Koppeleinrichtung ist ebenfalls, wie die feste Branche, einstückig mit dem Körperelement I der Gefäßklemme ausgeführt. Die beiden Greifelemente der Betätigungseinrichtung können durch eine Voreinstellung an den eingerasteten Klinken einen bestimmten Abstand zueinander einnehmen. Der Abstand der Greifelemente kann so vorgewählt sein, dass das Betätigungswerkzeug mit seiner Greifeinrichtung leicht auf die Haltebacken der Koppeleinrichtung geschoben werden kann. In dieser vorgewählten Stellung, sind die beiden Greifelemente in etwa parallel beabstandet. Diese Ausführung der Greifelemente und die Anordnung der Haltebacken ermöglicht somit ein leichtes Komplettieren der Gefäßklemme mit der Bedieneinrichtung. Jede Haltebacke der Koppeleinrichtung ist u-förmig ausgebildet, d.h., dass eine Haltebacke aus einer Anlagefläche und jeweils zwei Führungsrändern besteht, wobei die Führungsränder in Längsrichtung der Gefäßklemme und somit in Längsrichtung des Körperelementes verlaufen. Die Länge der Haltebacken spielt für die Führung der Greifelemente eine ebenso große Rolle wie der parallele Abstand der Haltebacken, um die Gefäßklemme sicher erfassen zu können. Auch die Höhe der Führungsränder an den Haltebacken, welche eine optimale Führung der Greifelemente unterstützen, ist zu beachten. Der Abstand der parallel beabstandeten Führungsränder ist gegenüber der Breite der Greifelemente abgestimmt, um ein einfaches aber sicheres und genaues Erfassen zu ermöglichen. Aufgrund der Ausführung der Haltebacken können die Greifelemente der Bedieneinrichtung nur in einer Richtung aufgeschoben oder erfasst werden, wodurch ein schnelles und sicheres Zusammenfügen bzw. Komplettieren der Gefäßklemme und der Bedieneinrichtung erfolgen kann. Die Ausführungsform der Greifelemente und der Koppeleinrichtung ermöglichen somit ein sicheres Handling des chirurgischen Instruments. Die optimale Führung der Greifelemente zwischen den Führungskanten der Haltebacken ermöglicht auch ein anschließendes schnelles und sicheres Einführen der Klinge eines Betätigungswerkzeuges in das Innensechskantprofil eines Verstellelementes.

Ein erstes Bauteil des erfinderischen Gegenstandes umfasst somit eine stufenlos fassende- und klemmende, stark verkürzte Gefäßklemme, ohne scherenartige Ausbildung und ohne sperriges Klinkensperrsystem, wobei die Gefäßklemme ein Körperelement, ein Zug- und Druckelement, ein Arbeitsmittel, eine Verstelleinrichtung, eine Koppeleinrichtung und eine Arretierungseinrichtung umfasst. Die vorteilhaft durch die Parallelführung der Haltebacken ausgeführte Koppeleinrichtung wird benötigt, um eine Zerleg- und Komplettierbarkeit des chirurgischen Instrumentes sicher und leicht zu ermöglichen. Vorteilhafterweise ist die Gefäßklemme aus korrosionsarmen Stahl, vorzugsweise aus Edelstahl, Titan oder Tantal hergestellt. Die Gefäßklemme, insbesondere eingesetzt als Aortenklemme bei laparoskopischen Operationen an der infrarenalen Aorta, gewährleistet, mit Hilfe einer Bedieneinrichtung und einem Betätigungswerkzeug, ein sicheres Abklemmen auch sklerotischer Gefäße. Der Drehverschluss mit Feingewinde erlaubt ein dosiertes Schließen und Öffnen der Gefäßklemme. Und durch den Einsatz von auswechselbaren Polstereinsätzen an den Branchen der erfindungsgemäßen Gefäßklemme wird ein atraumatisches Klemmen von Gefäßen ermöglicht.

Die Lösung der zweiten Aufgabe besteht darin, eine Bedieneinrichtung zu entwickeln, wobei die Bedieneinrichtung mehrere Aufgaben zu erfüllen hat. Eine Aufgabe besteht darin, dass die Bedieneinrichtung eine erfindungsgemäße Gefäßklemme erfassen, klemmen, führen und entsprechend wieder frei geben kann. Die zweite Aufgabe die von der Bedieneinrichtung zu erfüllen ist, besteht darin, ein Betätigungswerkzeug in etwa spielfrei aufzunehmen und ebenfalls zu führen. Ausgang der Entwicklung hierbei ist der aus dem Stand der Technik zu entnehmende Offenbarungsgehalt. Ein Offenbarungsgehalt, der sich beispielsweise aus den aufgeführten Dokumenten im Hintergrund der Stand der Technik, entnehmen lässt. Hier wären vor allem die Kocherklemme und deren Weiterentwicklungen, wie das aus der US 2003/0212435 A1 bekannte medizinische Instrument 600 zu nennen. Bei der Bedieneinrichtung aus dem vorgenannten US-Dokument, ist das Betätigungswerkzeug über viele Einzelteile fest mit der Bedieneinrichtung verbunden und behindert aufgrund der Größe das Gesichtsfeld des Chirurgen. Basis der erfinderischen Bedieneinrichtung ist somit eine bekannte Gefäßklemme, bestehend aus zwei, beweglich miteinander scherenartig verbundenen Schenkeln, die am distalen Ende eine Klemm- und Greifeinrichtung aufweisen, die zwei Greifelemente umfasst, und am proximalen Ende eine Griffeinrichtung aufweisen, die beispielsweise zwei ringförmige Griffe umfasst, sowie nahe der ringförmigen Griffe eine Feststellvorrichtung aufweisen, die zwei, Rastzähne aufweisende Klinken am Schenkel umfasst, mit der die Greifelemente in einer von mehreren Stellungen eingestellt werden können. Die Aufgaben der erfindungsgemäßen Bedieneinrichtung unterscheiden sich wesentlich von den Aufgaben einer Gefäßklemme. Ein Erfassen und dosiertes Abklemmen eines Gefäßes, mit Hilfe einer stufenlosen Verstelleinrichtung, wird nicht benötigt. Die Lösung besteht daher in einer Bedieneinrichtung zum Erfassen, Klemmen und wieder Lösen einer separaten, wie zuvor aufgezeigt, erfinderischen Gefäßklemme. Daher besteht die Bedieneinrichtung aus zwei beweglichen, miteinander scherenartig verbundenen Schenkeln, die am distalen Ende als Greifelemente ausgebildet sind und am proximalen Ende mit Griffelementen, sowie nahe der Griffelemente mit einer, Rastzähne aufweisenden Feststellvorrichtung, versehen sind. Ein Schenkel der Bedieneinrichtung ist daher Träger eines daran angeordneten Führungselementes, wobei das Führungselement am Schenkel befestigt ist. Das Führungselement dient einem Betätigungswerkzeug zur Aufnahme und Führung. Das eine freie Ende des Führungselementes mündet direkt vor der Verstelleinrichtung der Gefäßklemme, wodurch die Klinge eines eingeführten Betätigungswerkzeuges unmittelbar in ein Verstellelement der Verstelleinrichtung eingreifen kann. Das entgegengesetzte freie Ende des Führungselementes verläuft zwischen den beiden ringförmigen Griffelementen und endet ungefähr am Ende der ringförmigen Griffelemente. Die Greifeinrichtung besteht aus zwei Greifelementen, welche in etwa parallel zueinander beabstandet sind. Die Greifelemente weisen in etwa eine u-förmige Ausgestaltung auf, wobei die beiden Schenkel des "U" durch die beiden Greifelemente gebildet werden. Im Scheitelpunkt der u-förmigen Ausgestaltung sind die Greifelemente gelenkartig miteinander verbunden. Ist die Klemme im Bereich der Griffelemente weit geöffnet, sind die beiden u-förmigen Schenkel der Greifelemente ebenfalls geöffnet und bilden in etwa ein großes "U", dessen Schenkel an den freien Enden voneinander weg, bzw. auseinander weisen. Werden die Griffelemente zum Schließen der Greifelemente aufeinander zu bewegt, bis die beiden, an den Schenkeln der Klemme befindlichen Klinken sich berühren, nähern sich die freien Enden der beiden Schenkel ebenfalls an, bzw. bewegen sich aufeinander zu. In dieser Stellung sind die beiden Greifelemente parallel zueinander beabstandet. Der Abstand weist einen bestimmten Betrag auf. Der Betrag entspricht in etwa dem korrespondierenden Betrag, der durch den Koppelbereich an der erfinderischen Gefäßklemme vorgegeben wird. Der Betrag des Koppelbereiches entspricht wiederum dem Maß, welches sich aus der Breite des Körperelementes und der beiden Wandstärken der Haltebacken des Koppelbereiches ergibt. Ein weiteres Aufeinanderzubewegen der Griffelemente bewirkt, dass die zwei, sich mit Rastzähnen gegenüberstehenden Klinken, zum Arretieren ineinander greifen können. Aufgrund der zusammenwirkenden Zähne kann die Feststellvorrichtung in einer von mehreren einzelnen Stellungen eingestellt werden. Damit kann die aufzubringende Kraft, zum Klemmen einer Gefäßklemme in der Greifeinrichtung einer Bedieneinrichtung, unterschiedlich sein.

Das zweite Bauteil des erfinderischen Gegenstandes umfasst damit eine Bedieneinrichtung, die scherenartig ausgebildet ist und zusätzlich zu den bekannten ringförmigen Griffen und einer bekannten Feststelleinrichtung über zwei Greifelemente verfügt, welche in ihrer Ausgestaltung derart geformt sind, dass diese einen Koppelbereich einer erfindungsgemäßen Gefäßklemme erfassen und klemmen können. Zur Bedienung der, an einer Gefäßklemme angeordneten Verstelleinrichtung, verfügt die Bedieneinrichtung über ein Führungselement, welches das Betätigungswerkzeug aufnehmen kann. Vorteilhafterweise ist die Bedieneinrichtung aus korrosionsarmen Stahl, vorzugsweise aus Edelstahl, Titan oder Tantal hergestellt. Die Bedieneinrichtung gewährleistet bei der Operation ein sicheres Handling einer Gefäßklemme und bewirkt, aufgrund des Kopplungseffektes zwischen der Gefäßklemme und dem Bedienelement, eine Zerleg- und Komplettierbarkeit des chirurgischen Instruments. Mit anderen Worten ausgedrückt handelt es sich bei der Entwicklung der erfinderischen Bedieneinrichtung um eine medizinische Zange, die als greifende- und haltende Zange in Sonderform ausgebildet ist. Bei dieser erfindungsgemäßen medizinischen Zange der Bedieneinrichtung handelt es sich im Prinzip um ein zweischenkliges Werkzeug, welches aus zwei Griffen (Griffelemente), einem Gelenk und einem Zangenkopf (Greifeinrichtung), der nach dem Hebelprinzip funktioniert, gebildet ist. Hierbei sind zwei zweiseitige Hebel miteinander durch ein Gelenk verbunden, wobei die Schenkel, an denen die Griffelemente angeordnet sind, die längeren Hebelarme bilden und die Schenkel des Zangenkopfes (Greifeinrichtung) die kürzeren Hebelarme bilden. Nach dem Hebelgesetz wird die, auf die Griffelemente aufgebrachte Handkraft verstärkt und mittels Zangenkopf (Greifeinrichtung) auf die Koppeleinrichtung übertragen. Der Zangenkopf (Greifeinrichtung) weist zwei Greifzangen (Greifelemente) auf, wobei die Backen des Zangenkopfes bzw. die Arbeitsflächen der Greifelemente einer Flachzange nachempfunden wurden. Im geschlossen Zustand des Zangenkopfes und somit der Greifeinrichtung der Bedieneinrichtung bilden die beiden Greifelemente ein geöffnetes Maul. Die Maulöffnung ist im geschlossenen Zustand der Greifeinrichtung kleiner als die Breite der Koppeleinrichtung. Befindet sich die Greifeinrichtung im geöffneten Zustand, ist die Maulöffnung wesentlich größer als die Breite der Koppeleinrichtung.

Die Lösung der dritten Aufgabe besteht darin, ein Betätigungswerkzeug zu entwickeln, welches geeignet ist, auf eine Verstelleinrichtung einer erfinderischen Gefäßklemme einzuwirken. Die Verstelleinrichtung wurde bereits bei der Lösung der ersten Aufgabe erläutert. Auch zu der notwendigen Führung des Betätigungswerkzeuges an der Bedieneinrichtung gibt es in der Lösung der zweiten Aufgabe entsprechende Angaben. Das Betätigungselement hat, wie die zwei zuvor beschriebenen erfinderischen Gegenstände, das Erfordernis der Zerleg- und Komplettierbarkeit zu erfüllen. Die aus dem Stand der Technik bekannten Gefäßklemmen sind aber einstückig ausgeführt. Auch die bekannten Aortenklemmen des Unternehmens Eisold-Instruments und aus der DE 20 2007 016 057 U1, welche ein Betätigungselement in Form eines drehbaren Handgriffes aufweisen, sind nicht an der Gefäßklemme und auch nicht an den zu verstellenden Branchen, lösbar und wiederverwendbar verbunden. Das Betätigungswerkzeug weist daher keine Verbindungselemente mit der Gefäßklemme und der Bedieneinrichtung auf, welche einer Zerlegbar- und Komplettierbarkeit des chirurgischen Instruments, im Wege stehen. Das Betätigungswerkzeug umfasst daher eine zylindrische Führungsstange, die der korrespondierenden Bohrung im Führungselement angepasst ist. An dem einen Ende der Führungsstange befindet sich ein Handgriff und am dem anderen freien Ende ist eine Klinge angeordnet. Der Durchmesser der Klinge darf den Durchmesser der Bohrung im Hohlzylinder des Führungselementes nicht überschreiten, sondern muss mindestens geringfügig kleiner sein, um ein leichtes Durchschieben der Klinge zu ermöglichen. Die Klinge kann aus unterschiedlich bekannten Außenprofilen gebildet sein. Das verwendete Außenprofil am Betätigungswerkzeug ist abhängig von dem verwendeten Profil des Formelementes im Verstellelement der Verstelleinrichtung an der Gefäßklemme. Wird im Verstellelement ein Schlitz zur Betätigung einer Drehbewegung vorgesehen, so weist das Betätigungswerkzeug eine Schraubendreherklinge auf Weist der Schraubenkopf des Verstellelementes einen Kreuzschlitz oder ein Torxprofil auf, so ist die Klinge des Betätigungswerkzeuges entsprechend angepasst. Vorzugsweise wird im Schraubenkopf des Verstellelementes ein Innensechskant verwendet, worauf die Führungsstange mit einem korrespondierenden Außensechskant ausgebildet wird. Dabei gilt zu beachten, dass die Klinge nicht größer sein darf als der Durchmesser der zylindrischen Führungsstange, weil die Klinge ebenfalls durch das Führungselement hindurch geführt werden muss.

Das dritte Bauteil des erfinderischen Gegenstandes umfasst damit ein Betätigungswerkzeug, das über eine zylindrische Führungsstange verfügt, die mit einer daran angeordneten Klinge durch die Bohrung eines Führungselementes, angeordnet an der Bedieneinrichtung, geführt werden kann und einen Handgriff aufweist. Der Handgriff ermöglicht es, Drehbewegungen auszuführen, die eine Verstellung des Verstellelement und somit eine Verstellung der Branchen nach sich zieht. Vorteilhafterweise ist die Betätigungseinrichtung aus korrosionsarmem Stahl, vorzugsweise aus Edelstahl, Titan oder Tantal hergestellt. Das Betätigungswerkzeug gewährleistet bei der Operation ein sicheres Öffnen und Schließen der Branchen einer Gefäßklemme, sowie eine Zerleg- und Komplettierbarkeit des chirurgischen Instruments.

Es ist festzuhalten, dass das erfinderische chirurgische Instrument ganz allgemein bei einer großen Vielzahl verschiedener Zangen und anderer Klemmen mit geraden oder gekrümmten Klemmschenkeln, bzw. Branchen verschiedener Abmessungen, abhängig von den speziellen Anwendungsfällen, für welche sie vorgesehen sind, angewandt werden kann. D.h., jede Zange oder Klemme, welche mit einer erfindungsgemäßen Koppeleinrichtung ausgebildet wird, kann mit einer zugehörigen Bedieneinrichtung und Betätigungswerkzeug zu einem zerleg- und komplettierbaren Instrument umgestaltet werden.

Ein Ausführungsbeispiel der Erfindung ist in den Zeichnungen rein schematisch dargestellt und wird nachfolgend näher beschrieben. Es zeigt
- Figur 1: eine perspektivische Ansicht eines komplettierten chirurgischen Instruments, gemäß der vorliegenden Erfindung, und
- Figur 2: eine Ausführungsform einer erfindungsgemäßen Gefäßklemme in Seitenansicht, gemäß der vorliegenden Erfindung, und
- Figur 2a: zeigt eine Ausführungsform eines erfindungsgemäßen Greifelementes im Querschnitt, und

- Figur 3: eine Ausführungsform einer erfindungsgemäßen Gefäßklemme im Schnitt, gemäß der Figur 2 und gemäß der vorliegenden Erfindung, und
- Figur 4: eine Ausführungsform einer erfindungsgemäßen Gefäßklemme in Frontansicht, und
- Figur 4a: zeigt eine Ausführungsform einer erfindungsgemäßen Haltebacke im Querschnitt.

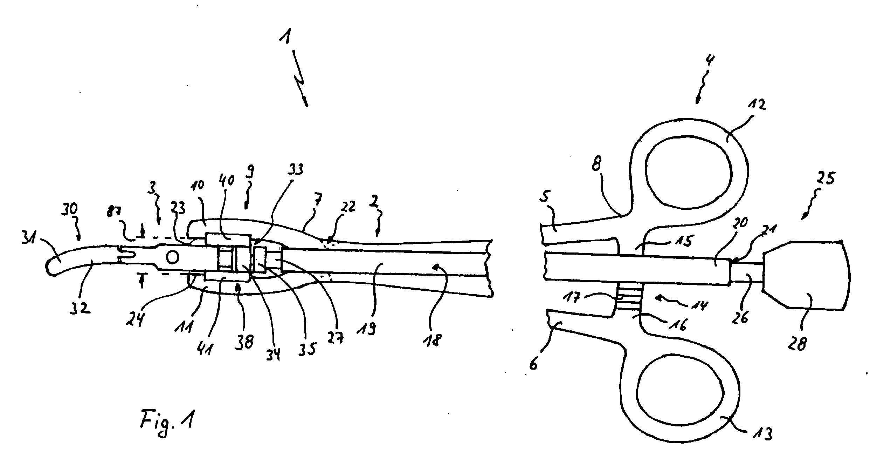

Das in der Fig.1 in prinzipieller Darstellung aufgezeigte, erfinderische dreiteilige medizinische Instrument 1, besteht im Wesentlichen aus einer erfinderischen Bedieneinrichtung 2, einer erfinderischen Gefäßklemme 3 und einem erfinderischen Betätigungswerkzeug 25. Das erfindungsgemäße chirurgische Instrument 1 entspricht den Vorschriften des Medizinproduktgesetzes und ist, aufgrund der verwendeten Materialen, bei der Reinigung für den Einsatz zur Sterilisation geeignet.

Die erfinderische Bedieneinrichtung 2 weist zwei bewegliche, bzw. gelenkig miteinander scherenartig verbundene Schenkel 5, 6 auf, die am distalen Ende 7 mit einer Greifeinrichtung 9, bestehend aus zwei Greifelementen 10, 11, und am proximalen Ende 8 mit einer Betätigungseinrichtung 4, bestehend aus zwei Griffelementen 12, 13, sowie, nahe der Griffelemente 12, 13, mit einer, Rastzähne 17 aufweisenden Feststellvorrichtung 14, bestehend aus zwei Klinken 15, 16, ausgebildet ist. Die Griffelemente 12, 13 können unterschiedliche Formen bekannter Ausbildung aufweisen, vorzugsweise sind die Griffelemente 12, 13 aber als Ringgriffe ausgebildet.

Der eine Schenkel 5 der Bedieneinrichtung 2 ist Träger eines daran angeordneten Führungselementes 18. Das Führungselement 18 kann einerseits am distalen Ende 7 des Schenkels 5 sowie andererseits, zum proximalen Ende 8 hin, auf der Klinke 15 des Schenkels 5 befestigt sein. Das Führungselement 18 bildet einen Hohlzylinder 19, dessen Länge 20 in etwa der Gesamtlänge eines Schenkels 5 mit Griffelement 12 einer Bedieneinrichtung 2 entspricht, und weist eine durchgehende Bohrung 21 auf, die der Aufnahme und Führung eines Betätigungswerkzeuges 25 dient. Der Hohlzylinder 19 besteht aus einem korrosionsarmen metallischen Rohr, vorzugsweise aus Edelstahl und ist mit Hilfe eines thermischen Verfahrens, vorzugsweise Laserschweißen, in der Nähe des Gelenkes 22 am Schenkel 5 und auf der Klinke 15 befestigt. Alternativ kann die Befestigung des Führungselementes 18 durch Laserschweißen nur auf der am Schenkel 5 angeordneten Klinke 15, oder nur auf dem Schenkel 5, erfolgen.

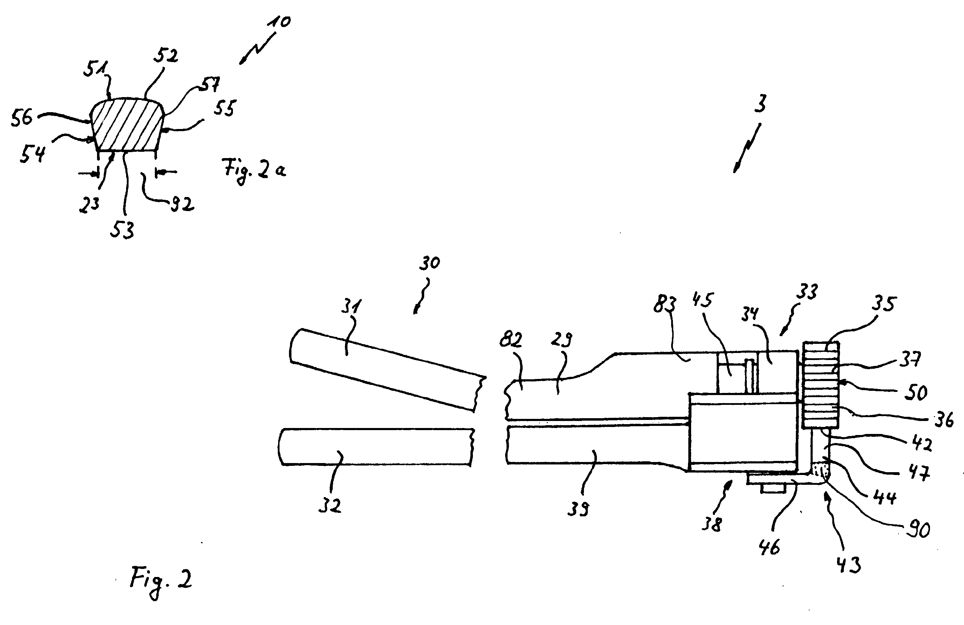

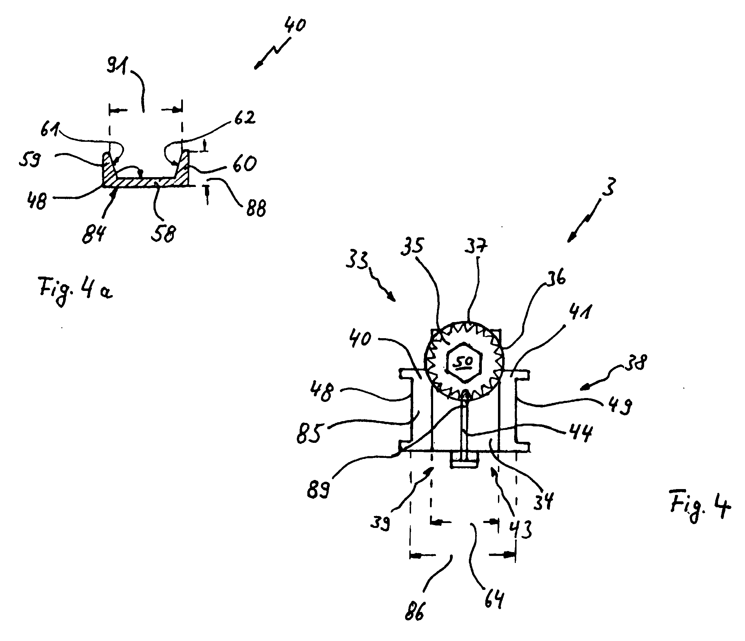

Die Greifeinrichtung 9 besteht aus zwei Greifelementen 10, 11, welche in etwa parallel zueinander beabstandet sind. Die Greifelemente 10, 11 weisen in der Parallelstellung eine in etwa u-förmige Ausgestaltung auf, wobei die beiden Längsschenkel des "U" durch die beiden Greifelemente 10, 11 gebildet werden. Wird die Bedieneinrichtung 2 an der Betätigungseinrichtung 4 geöffnet, öffnen sich auch die Greifelemente 10, 11 und die Öffnung des "U" erweitert sich. Beim Schließen der Betätigungseinrichtung 4, d.h., wenn die Griffelemente 12, 13 aufeinander zu bewegt werden, bewegen sich die Längsschenkel des "U" und somit die beiden Greifelemente 10, 11 ebenfalls aufeinander zu. Das Aufeinander zubewegen der Greifelemente 10, 11, kann nur soweit erfolgen, bis sich die Öffnung des "U" zwischen den beiden Längsschenkeln und somit zwischen den beiden Greifelementen 10, 11 um einen bestimmten Betrag etwas verkleinert hat. Dieser Betrag der Verkleinerung der Öffnung im "U" entspricht der Kraft, welche durch das Bedienelement 2 auf die parallel beabstandeten Haltebacken 40, 41 (siehe Figur 4) der Koppeleinrichtung 38 aufgebracht werden kann, um die Gefäßklemme 3 sicher zu erfassen und zu halten. Ist eine Bedieneinrichtung 2 mit einer Gefäßklemme 3 gekoppelt, können die Greifelemente 10, 11 aber maximal eine parallele Stellung, bzw. Abstand 87 zueinander einnehmen. Die Stellung der Greifelemente 10, 11 wird aufgrund des parallelen Abstandes der beiden Haltebacken 40, 41 der Koppeleinrichtung 38, bestimmt. Im Scheitelpunkt (des Gelenkes 22) der u-förmigen Ausgestaltung, sind die Greifelemente 10, 11 gelenkartig miteinander verbunden. Gemäß der Figur 2a weisen die innen liegenden Arbeitsflächen 23, 24 der Greifelemente 10, 11 eine strukturierte beispielsweise in Form einer Verzahnung. Die Struktur der Oberfläche kann ein genutetes-, ein gezahntes-, ein perforiertes- oder gerändeltes Profil aufweisen. Aufgrund der strukturierten Arbeitsflächen 23, 24 wird eine zuverlässige Klemmung zwischen der Gefäßklemme 3 und der Bedieneinrichtung 2 sicher gestellt. Die Form eines Greifelementes 10, 11, bzw. die Form der Backen eines Greifelementes 10, 11, ist annähernd der Form der Backen einer Flachzange nachempfunden. Die Greifelemente 10, 11 sind länglich und zum Ende hin verjüngend ausgeführt, wobei der Querschnitt eines Greifelementes 10,11 weitestgehend einem Quadrat entspricht, welches auf einer Seite einen Kreisabschnitt aufweist, wodurch diese Seite des Greifelementes 10,11 eine ballige Außenseite 51 erhält. Diese ballige Außenseite 51 eines Greifelementes 10, 11 ist aufgrund der Verletzungsgefahr des Patienten unabdingbar. Die dem Kreisabschnitt bzw. der balligen Fläche 52 der Außenseite 51 gegenüberliegende Seite entspricht der Arbeitsfläche 23, 24, die einerseits eben ist und andererseits eine Verzahnung 53 aufweist. Die beiden, sich an die Arbeitsfläche 23, 24 um jeweils 90 Grad anschließenden und parallel beabstandeten Seitenflächen 54, 55, bilden die Führungsseiten 56, 57 der Greifelemente 10, 11. Vorteilhafterweise können die Seitenflächen 54, 55 eine verjüngende Neigung von wenigen Graden in Richtung der Arbeitsfläche 23, 24 hin, aufweisen, wodurch der Querschnitt für ein Greifelement 10, 11 annähernd einem auf dem Kopf stehenden Trapez, entspricht. Korrespondierend zu den geneigten Seitenflächen 54, 55 der Greifelemente 10, 11, weisen die innen liegenden Führungsflächen 61, 62 der Haltebacken 40, 41 ebenfalls eine Neigung auf. Die Neigung entspricht einer verjüngenden Neigung der innen liegenden Führungsflächen 61, 62 zur Anlagefläche 48, 49 der Grundwand 58 hin. Die Führungsseiten 56, 57 der Greifelemente 10, 11 übernehmen mit den innen liegenden Führungsflächen 61, 62 der Stege 59, 60 (siehe hierzu Figur 4a) die Führung und Zentrierung der Gefäßklemme 3. Aufgrund der guten Zentrierung der Gefäßklemme 3 durch das Bedienelement 2, stehen die beiden Bauteile optimal zueinander, wodurch das Betätigungswerkzeug 25 mit dem Verstellelement 35 eine fluchtende Verbindung eingehen kann. Die relativ lang am Körperelement 39 ausgeführten Haltebacken 40, 41 und die an den Haltebacken 40, 41 und Greifelementen 10, 11 angeordneten Führungselemente, verhindern nach ihrem Zusammenfügen ein Abkippen der Gefäßklemme 3 in der Koppeleinrichtung 38. Ein Abkippen der Gefäßklemme 3 zwischen den Greifelementen 10, 11 der Bedieneinrichtung 2 ist somit nicht möglich. Diese vorteilhafte Ausführungsform der Koppeleinrichtung 38 und die vorteilhafte Ausführungsform der Greifeinrichtung 9 ist ein wichtiges Kriterium des sicheren Handlings bei der Anwendung des erfinderischen chirurgischen Instruments 1.

Aus der Figur 1 ist weiterhin ersichtlich, dass die Betätigungseinrichtung 4 sich in einer arretierten Stellung befindet. In der Arretierungsstellung wirken die Rastzähne 17 der Klinken 15, 16 zusammen, und die Greifelemente 10, 11 der Greifeinrichtung 9 umfassen im Bereich der Koppeleinrichtung 38 eine Gefäßklemme 3. Die Gefäßklemme 3 umfasst ein Arbeitsmittel 30, eine Verstelleinrichtung 33 und eine Koppeleinrichtung 38. Das Arbeitsmittel 30 wird aus zwei Branchen 31, 32, die Verstelleinrichtung 33 aus einer Halterung 34 und einem Verstellelement 35 gebildet. Die Koppeleinrichtung 38 besteht aus zwei Haltebacken 40, 41, die seitlich im Bereich der Verstelleinrichtung 33 angeordnet sind. Weitere Angaben zu der erfinderischen Gefäßklemme 3 sind der Figur 2, 3 und der Figur 4 zu entnehmen. Die Greifelemente 10, 11 der Greifeinrichtung 9 haben die Gefäßklemme 3, an den Haltebacken 40, 41 der Koppeleinrichtung 38, zwischen sich fest eingeklemmt. Die Branchen 31, 32 der Gefäßklemme 3 sind ebenfalls geschlossen. Zum Öffnen der Branchen 31, 32 ist ein Betätigungswerkzeug 25 zu benutzen.

Das Betätigungswerkzeug 25 besteht aus einer zylindrischen Führungsstange 26, einer Klinge 27 und einem Handgriff 28. Die Länge der zylindrischen Führungsstange 26 ist um einiges länger als die Länge des Hohlzylinders 19 und der Durchmesser der zylindrischen Führungsstange 26 ist der korrespondierenden Bohrung 21 des Hohlzylinders 19 angepasst und entspricht in etwa einigen Millimetern, vorzugsweise 5 mm. An dem einen Ende der zylindrischen Führungsstange 26 ist ein ergonomisch gestalteter Handgriff 28, zum Drehen des Betätigungswerkzeuges 25, angeordnet. Der Handgriff 28 besteht aus einem metallischen Hohlkörper, der, wie das gesamte Betätigungswerkzeug 25, im Reinigungsprozess sterilisationstauglich ist. Am anderen Ende der zylindrischen Führungsstange 26 befindet sich eine Klinge 27. Die Klinge 27 kann verschiedenartige bekannte Werkzeug-Ausgestaltungen aufweisen. Beispielsweise in Form einer geraden Klinge, einer Kreuzschlitzklinge, einer Inbusklinge oder einer Torxklinge. Vorzugsweise wird die Form eines Außenvielkantes, insbesondere für eine Innensechskantschraube, eingesetzt. In der bevorzugten Ausführung bildet somit das Betätigungswerkzeug 25 einen speziellen Schraubendreher mit Inbusprofil für ein chirurgisches Instrument 1. Die Klinge 27 kann an der zylindrischen Führungsstange 26 befestigt oder vorteilhafterweise einstückig mit dieser verbunden sein. Die Größe der Klinge 27 entspricht maximal der Größe der Bohrung 21 im Hohlzylinder 19, ist aber vorteilhafterweise etwas kleiner ausgelegt.

Im vorliegenden Beispiel der Figur 1 ist der dargestellte erfinderische Gegenstand ein komplettiertes, aus drei Teilen bestehendes, chirurgisches Instrument 1, vorzugsweise in der Ausführung einer Aortenklemme. Das dreiteilige chirurgische Instrument 1 kann zwischen der Gefäßklemme 3, der Bedieneinrichtung 2 und dem Betätigungswerkzeug 25, wie zuvor beschrieben, auf einfache Art und Weise, wiederholbar getrennt, bzw, zerlegt und komplettiert werden. Das Bauteil der Gefäßklemme 3 wird als einfaches zangenförmiges chirurgisches Instrument 1 verwendet, das einen mechanischen Vorgang zwischen seinen Branchen 31, 32 dazu benutzt, Gewebe oder Gefäße zusammenzudrücken und zu klammern. Das Klammern erfolgt durch den Druck der Branchen 31, 32, welcher durch die Drehbewegung des Verstellelementes 35 erzeugt wird. Zur Erzeugung der Drehbewegung an dem Verstellelement 35, wird ein Betätigungswerkzeug 25 eingesetzt. Nach jeder Drehbewegung am Betätigungswerkzeug 25 ist eine Sperrwirkung für die Branchen 31, 32 vorhanden, siehe hierzu die Erläuterungen in der Figur 2.

Die Fig.2 zeigt skizzenhaft eine Ausführungsform einer erfindungsgemäßen Gefäßklemme 3 in Seitenansicht und die Fig.2a zeigt ein Greifelement 10, 11 aus der Figur 1 im Querschnitt.

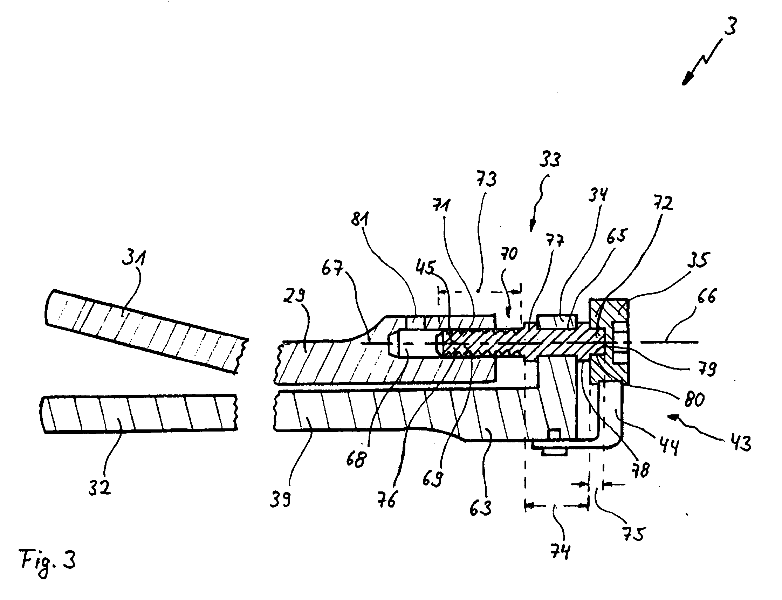

Die Gefäßklemme 3 umfasst als Basis ein Körperelement 39 und besteht im Wesentlichen aus einem Arbeitsmittel 30, einer Verstelleinrichtung 33, einer Arretierungseinrichtung 43 und einer Koppeleinrichtung 38. Das Arbeitsmittel 30 wird aus zwei Branchen 31, 32 gebildet, wobei die eine Branche 32, gegenüber ihrem freien Ende, einstückig aus dem einen Ende des Körperelementes 39 hervorgeht und fest damit verbunden ist. Die andere Branche 31 hingegen ist über einen Stift (nicht dargestellt) mit dem Zug- und Druckelement 29 verbunden und beweglich um einen Stift des Zug- und Druckelements 29 angeordnet. Am anderen Ende des Körperelementes 39, der Branche 32 abgewandten Seite gegenüber, befindet sich eine, einstückig aus dem Körperelement 39 hervorstehende Halterung 34. Die Halterung 34 bildet mit dem Verstellelement 35 eine Verstelleinrichtung 33. Die Halterung 34 ist dabei senkrecht zur Längsachse des Körperelements 39 und senkrecht zur Längsachse des Zug- und Druckelementes 29 angeordnet, wobei die Halterung 34 aus einem geometrischen Körper gebildet ist, vorzugsweise aus einem rechteckigen Stab besteht, der im Winkel von 90 Grad zum Körperelement 39 an diesem angeordnet ist. Die stabförmige Halterung 34 bildet mit dem Körperelement 39 eine L-förmige Anordnung und nimmt das Verstellelement 35 auf. Einige nachstehende Bezugszeichen können der Figur 3 entnommen werden. Die Halterung 34 enthält dazu eine Öffnung 65, vorzugsweise ausgebildet als Bohrung. Die Öffnung 65 ist derart in der Halterung 34 angeordnet, dass die Mittellinie 66 der Öffnung 65 mit der Mittellinie 67 der Längsachse des Zug- und Druckelementes 29 fluchtet. Das Zug- und Druckelement 29 ist am distalen Ende 82 drehbar über einen Stift II (nicht dargestellt), der in der beweglichen Branche 31 angeordnet ist, mit dieser verbunden und am proximalen Ende 83 mit dem Verstellelement 35. Durch diese Anordnung der beiden Verbindungen an den beiden freien Enden des Zug- und Druckelementes 29, wird das Zug- und Druckelement 29 parallel zum Körperelement 39 angeordnet und kann auf der Oberfläche des Körperelementes 39 gleiten. Wenn das Verstellelement 35 gedreht wird, verschiebt sich das Zug- und Druckelement 29 axial gegen das Körperelement 39, wobei der Stift II am distalen Ende 82 des Zug- und Druckelementes 29 ebenfalls eine Bewegung ausführt, bzw. auf einer Kreisbahn verschoben wird. Durch die Verschiebung des Zug- und Druckelementes 29 kann somit die bewegliche Branche 31 geöffnet oder geschlossen werden. Die Verschiebung erfolgt durch das Drehen am Verstellelement 35. Das Drehen am Verstellelement 35 bewirkt eine stufenlose Verstellung der Branche 31. Dazu weist das Verstellelement 35 ein Innensechskantprofil 50 auf. Das Innensechskantprofil 50 nimmt zur Verstellung der Branche 31 ein Betätigungswerkzeug 25 (siehe Figur 1) auf. Mit dem Betätigungswerkzeug 25 wird eine entfernte Bedienung einer erfinderischen Gefäßklemme 3, bzw. deren Branche 31, ermöglicht. Die stufenlose Verstellung der Branche 31 wird über ein Feingewinde 76 (siehe Figur 3), erreicht, welches, wie bereits zuvor erläutert, am Verstellelement 35 angeordnet ist und das Verstellelement 35 mit dem Zug- und Druckelement 29 verbindet. Die Ausführungsform der Verbindung zwischen dem Zug- und Druckelement 29 und dem Verstellelement 35 kann der Figur 3 entnommen werden.

Das Verstellelement 35 ist im Prinzip aus zwei Teilen gefertigt, einen Schraubenkopfteil 36 und einen Schraubenbolzenteil 45. Beide Teile werden durch eine Montage zu einem festen Teil, einem unlösbaren Verstellelement 35, zusammengefügt In einer anderen Ausführung sind der Schraubenkopfteil 36 und der Schraubenbolzenteil 45 des Verstellelementes 35 einstückig ausgeführt. Zwischen dem Schraubenkopfteil 36 und dem Schraubenbolzenteil 45 befindet sich die Halterung 34 der Verstelleinrichtung 33. Das Schraubenbolzenteil 45 weist mindestens einen Flansch I 77, II 78 und ein Feingewinde 76 (siehe Figur 3) auf, womit eine selbsthemmende Wirkung in der Gewindebohrung 68 des Zug- und Druckelementes 29 erreicht wird. Der Schraubenbolzenteil 45 greift somit in das Zug- und Druckelement 29 ein. Das Schraubenkopfteil 36 des Verstellelementes 35 weist einen Innensechskant 50 (siehe Figur 4) und am Umfang ein Rastprofil 37 auf. Das Rastprofil 37 besteht aus Querrillen, vorzugsweise Rillen in Form einer Evolventenverzahnung, in welche eine Rastfeder 44 eingreift. Die Rastfeder 44 ist unterhalb des Körperelementes 39 angeordnet. Die Befestigung der Rastfeder 44 am Körperelement 39 kann auf verschiedene Art und Weise erfolgen. Vorteilhafterweise wird die Rastfeder 44 angenietet, auch eine Schraubbefestigung ist denkbar. Die Rastfeder 44 ist derart gestaltet, dass diese aus zwei Schenkeln I, II 46, 47 gebildet wird. Die beiden Schenkel I, II 46, 47 sind winklig zueinander angeordnet und bilden einen Winkel in etwa von 90 Grad. Damit steht der vertikale Schenkel II 47 der Rastfeder 44 parallel beabstandet zur Halterung 34, wobei die dünne Eingreifkante 42 der Rastfeder 44 ebenfalls parallel zur Halterung 34 verläuft. In dieser Stellung kann die Eingreifkante 42 des vertikalen Schenkels II 47 nicht in die Querrillen des Rastprofils 37 eingreifen. Daher ist es notwendig, den vertikalen Schenkel II 47 um 90 Grad zu drehen. Durch eine 90 Grad Drehung bleibt der parallele Abstand des vertikalen Schenkels II 47 zur Halterung 34 erhalten, aber die Eingreifkante 42 nimmt eine andere Stellung ein. Die Drehung des vertikalen Schenkels II 47 wird unmittelbar in der Nähe des Winkels, der zwischen den beiden Schenkeln I 46, II 47 gebildet ist, durch Verdrillen des vertikalen Schenkels 47, vorgenommen. Nach der Verdrillung 90 verläuft die Eingreifkante 42 des vertikalen Schenkels II 47 parallel zu den Querrillen 37 im Verstellelement 35. Der Schenkel I 46 hingegen bildet den horizontalen Befestigungsschenkel I 46, und der Schenkel II 47, der den vertikalen beweglichen Schenkel II 47 bildet, kann in zwei Richtungen ausgelenkt werden. Die Auslenkung ist relativ kurz und kann im Uhrzeigersinn oder entgegen dem Uhrzeigersinn erfolgen, je nachdem, welche Drehrichtung am Verstellelement 35, mit Hilfe des Betätigungswerkzeuges 25, zum Öffnen und Schließen der Branchen 31, 32, benötigt wird. Die Rastfeder 44 bildet in Kombination mit dem Verstellelement 35 eine Arretierungseinrichtung 43.

Die Fig.3 zeigt im Schnitt eine Ausführungsform einer erfindungsgemäßen Gefäßklemme 3. Aus der Schnittdarstellung ist das tragende Körperelement 39 mit den erfindungsgemäßen Ausführungen der Verstelleinrichtung 33 und der Arretierungseinrichtung 43 ersichtlich. Die in der Figur 2 aufgezeigten Bezugszeichen werden hier analog übernommen, aber nicht näher beschrieben.

Am proximalen Ende 63 des Körperelementes 39 ist die, auf dem Körperelement 39 angeordnete Halterung 34, gut ersichtlich. Die Halterung 34 bildet einen geometrischen Körper, vorzugsweise einen quaderförmigen Körper. Der quaderförmige Körper entspricht einem rechteckigen Stab, der im Winkel von 90 Grad zum Körperelement 39 an diesem angeordnet ist. Die stabförmige Halterung 34 bildet mit dem Körperelement 39 eine L-förmige Anordnung. Die Halterung 34 weist die gleiche Breite 64 (siehe hierzu Figur 4) wie das feste Körperelement 39 auf und steht senkrecht auf der Längsachse des Körperelementes 39 und senkrecht zur Längsachse des Zug-und Druckelementes 29. Die Halterung 34 enthält eine Öffnung 65, vorzugsweise ausgebildet als Bohrung. Die Bohrung 65 ist derart in der Halterung 34 angeordnet, dass die Mittellinie 66 der Öffnung 65 mit der Mittellinie 67 der Längsachse des Zug- und Druckelementes 29 fluchtet. D.h., die Mittellinie 66 der Bohrung 65 verläuft parallel beabstandet zur Mittellinie des Körperelementes 39 und auf der gleichen Mittellinie 67 der Gewindebohrung 68 im Zug- und Druckelement 29.

Die Bohrung 65 dient der Aufnahme und Führung einer Antriebswelle 69, die rechts und links aus der Halterung 34 hervorsteht. Die Antriebswelle 69 stellt im Prinzip das Verbindungselement 70 zwischen dem Zug- und Druckelement 29 und dem Verstellelement 35 dar. Die Antriebswelle 69 greift mit dem einen vorstehenden Wellenende I 71 in das verschiebbare Zug- und Druckelement 29 ein und nimmt mit dem anderen vorstehenden Wellenende II 72 ein Verstellelement 35 auf Dabei ist die Antriebswelle 69 zylindrisch ausgebildet und weist drei Funktionsbereiche I, II, III auf. Der Funktionsbereich I 73 umfasst das Wellenende I 71, welches als Schraubenbolzenteil 45 mit Feingewinde 76 ausgebildet ist und in die Gewindebohrung 68 des verschiebbaren Zug- und Druckelementes 29 eingreift. Der Funktionsbereich II 74 hingegen entspricht weitestgehend dem mittleren Teil der Antriebswelle 69, der aus zwei beabstandeten Flanschen I 77, II 78 gebildet wird, und welche die Halterung 34 zwischen sich aufnehmen. Nach der Konfektionierung der Antriebswelle 69 in die Bohrung 65 der Halterung 34 befindet sich die Halterung 34 zwischen den beiden auf der Antriebswelle 69 angeordneten Flanschen I 77 und II 78, wodurch die Antriebswelle 69 eine Führung erhält. Die Halterung 34 und die Flansche I 77, II 78 sind zueinander angepasst. Die Anpassung erfolgt gemäß einer DIN. Die DIN- Angaben enthalten einem Passmaß, beispielsweise für "H7" und entnehmbar aus einer Tabelle mit der Bezeichnung "Welle/Bohrung". Dieses eng tolerierte Passmaß ermöglicht, dass die Flansche 77, 78 der Antriebswelle 69 die Halterung 34 spielfrei zwischen sich führen. Bleibt noch der Funktionsbereich III 75, der dem anderen Wellenende II 72 entspricht und aus einem zylindrischen Zapfen 79 gebildet wird und ein Verstellelement 35 aufnehmen kann. Der zylindrische Zapfen 79 kann beispielsweise ebenfalls mit einem Gewinde ausgestattet sein. Das Verstellelement 35 weist daher in der zweiteiligen Ausführung, auf einer Seite eine Öffnung 80 auf, die in der Größe mit dem zylindrischen Zapfen 79 und dessen Ausführung korrespondiert. In der einteiligen Ausführung geht die Antriebswelle 69 aus dem Verstellelement 35 hervor, wodurch die Öffnung 80 im Verstellelement 35 entfällt.

Vorteilhaft aufzuzeigen ist noch eine Reinigungsöffnung 81, die am Ende der Gewindebohrung 68 im Zug- und Druckelement 29 angeordnet ist. Die Gewindebohrung 68 ist eine Sackbohrung und daher sehr schlecht zu reinigen. Um die Vorgaben der Sterilisationsbedingungen zu erfüllen, befindet sich am Ende der Sackbohrung eine Reinigungsöffnung 81, die mit der Gewindebohrung 68 in Verbindung steht. Die Reinigungsöffnung 81 ist auf der Oberseite des Zug- und Druckelementes 29 und senkrecht zur Mittellinie 67 der Gewindebohrung 68 angeordnet. Die Verstelleinrichtung 33 wurde bereits in der Figur 2 erwähnt und wird in der Figur 4 näher erläutert.

Fig. 4 zeigt eine Ausführungsform einer erfindungsgemäßen Gefäßklemme 3 in Frontansicht und Fig.4a zeigt eine Haltebacke 40, 41 aus der Koppeleinrichtung 38, welche an der Gefäßklemme 3 angeordnet ist.

Aus der Frontansicht der Figur 4 ist das Körperelement 39 mit der Koppeleinrichtung 38, der Verstelleinrichtung 33 und der Arretierungseinrichtung 43 ersichtlich. Die Koppeleinrichtung 38 besteht aus zwei Haltebacken 40, 41, die einstückig mit dem Körperelement 39 am proximalen Ende 63 (siehe Figur 3) der Gefäßklemme 3 verbunden und seitlich in Längsrichtung am Körperelement 39 angeordnet sind. Jede Haltebacke 40, 41 ist im Querschnittsprofil u-förmig ausgebildet (siehe Figur 4a) ist. Eine u-förmige Haltebacke 40, 41 wird aus einer Grundwand 58 und zwei parallel beabstandeten Stegen 59, 60 gebildet. Die Außenwand 84 der Grundwand 58 ist mit dem Körperelement 39 verbunden. D.h., das die Außenwand 84 die dem Körperelement . 39 zugewandte Seite ist, wobei die innen liegende Fläche die Anlagefläche 48, 49 für die Greifelemente 10, 11 bildet. Die freien Enden der Stege 59, 60 weisen vom Körperelement 39 weg. Die innen liegenden Flächen der Stege 59, 60 bilden die Führungsflächen 61, 62, die einen bestimmten Abstand 91 aufweisen, der mit dem Abstand der Führungsseiten 56, 57, der durch die Breite 92 (siehe Figur 2a) der Greifelemente 10, 11 vorgegeben ist, korrespondiert. Die innen liegende Fläche der Grundwand 58 bildet die Anlagefläche 48, 49. Vorteilhafterweise sind die beiden Anlageflächen 48, 49 der Haltebacken 40, 41 parallel beabstandet. Der parallele Abstand ergibt sich aus der Breite 64 des Körperelementes 39 und der Wanddicke 85 der beiden Haltebacken 40, 41, was einem bestimmten Betrag X bzw. eine bestimmte Breite 86 in mm für die Koppeleinrichtung 38 ergibt. Der Betrag X für die Koppeleinrichtung 38 liegt vorzugsweise in einem Bereich von 10 mm bis 20 mm, je nach Ausführung der Breite 64 des Körperelementes 39 einer Gefäßklemme 3. Bei diesem Betrag X in mm sind die Greifelemente 10, 11 der Greifeinrichtung 9 ebenfalls parallel beabstandet. D.h., der parallele Abstand 87 (siehe Figur 1) der Greifelemente 10, 11 ist der Breite 86 der Koppeleinrichtung 38 angepasst. Zur Verhinderung des Abgleitens der Greifelemente 10, 11 von den Anlageflächen 48, 49 der Haltebacken 40, 41, weisen die Anlageflächen 48, 49 eine Oberflächenstruktur auf. Die Struktur der Anlageflächen 48, 49 kann mit den innen liegenden Arbeitsflächen 23, 24 der Greifelemente 10, 11, korrespondieren, wodurch die Reibung zwischen den Greifelementen 10, 11 und den Haltebacken 40, 41 vergrößert wird.

In einer weiteren Ausführung (nicht dargestellt) der Führungselemente in der Koppeleinrichtung 38 und in der Greifeinrichtung 9 kommen nicht die Anlageflächen 48, 49 der Haltebacken 40, 41 mit den Arbeitsflächen 23, 24 der Greifelemente 10, 11 in Eingriff, sondern nur die Führungsflächen 61, 62 der Haltebacken 40, 41 mit den Führungsseiten 56, 57 der Greifelemente 10, 11. In dieser Ausführung sind die Stege 59, 60 der Haltebacken 40, 41 in der Höhe 88 wesentlich länger ausgeführt. Die Führungsflächen 61, 62 der Haltebacken 40, 41 und die Seitenflächen 54, 55 sind mit einer Oberflächenstruktur ausgestattet, um ein Abrutschen der Greifelemente 10, 11, bzw. der Bedieneinrichtung 2 aus der Koppeleinrichtung 38 zu verhindern und vor allem ein gezieltes Erfassen der Gefäßklemme 3 zu ermöglichen.

Von der Verstelleinrichtung 33 ist das Verstellelement 35 mit dem zahnförmigen Rastprofil 37 auf dem Umfang des Schraubenkopfes 36 gut ersichtlich. Zur Arretierung der Branchen 31, 32 greift die Rastfeder 44 mit ihrem freien Ende 89 in das Rastprofil 37 ein. Die Rastfeder 44 ist am Körperelement 39 unterhalb der Halterung 34 befestigt. Das Verstellelement 35 hingegen befindet sich auf dem einen Wellenende II 72 der Antriebswelle 69, die in der Halterung 34 geführt wird. Im Schraubenkopf 36 des Verstellelementes 35 ist das innen liegende Formelement, bestehend aus einem Innensechskant 50, zum Einführen der Klinge 27 des Betätigungswerkzeuges 25, ersichtlich.

### Bezugszeichenliste

- 1: Chirurgisches Instrument
- 2: Bedieneinrichtung (v.1)
- 3: Gefäßklemme (v.1)
- 4: Betätigungseinrichtung (v.2)
- 5: Schenkel (v.2)
- 6: Schenkel (v.2)
- 7: Distales Ende (v.2)
- 8: Proximales Ende (v.2)
- 9: Greifeinrichtung (v.2)
- 10: Greifelement (v.2)
- 11: Greifelement (v.2)
- 12: Griffelement (v.2, 5)
- 13: Griffelement (v.2, 5)
- 14: Feststellvorrichtung (v.2)
- 15: Klinke (v.2, 5)
- 16: Klinke (v.2, 6)
- 17: Rastzähne (15, 16)
- 18: Führungselement (v.2)
- 19: Hohlzylinder (v.18)
- 20: Länge (v.19)
- 21: Bohrung (v.19)
- 22: Gelenk (v.2, 5, 6)
- 23: Arbeitsfläche (v.10)
- 24: Arbeitsfläche (v.11)
- 25: Betätigungswerkzeug (v.1)
- 26: Zylind. Führungsstange (v.25)
- 27: Klinge (v.25)
- 28: Handgriff (v.25)
- 29: Zug-u. Druckelement (v.3)
- 30: Arbeitsmittel (v.3)

- 31: Branche (v.3)
- 32: Branche (v.3)
- 33: Verstelleinrichtung (v.3)
- 34: Halterung (v.33, 39)
- 35: Verstellelement (v.33, 43)
- 36: Schraubenkopfteil (v.35)
- 37: Rastprofil (v. 33, 35)
- 38: Koppeleinrichtung (v.3)
- 39: Körperelement (v.3)
- 40: Haltebacke (v.38)
- 41: Haltebacke (v.38)
- 42: Eingreifkante (v.44)
- 43: Arretierungseinrichtung (v.3)
- 44: Rastfeder (v.43)
- 45: Schraubenteil (v.33, 35)
- 46: Schenkel I (v.44)
- 47: Schenkel II (v.44)
- 48: Anlagefläche (v.40)
- 49: Anlagefläche (v.41)
- 50: Innensechskant (v.33)
- 51: Außenseite (v.10, 11)
- 52: Ballige Fläche (v.10, 11)
- 53: Verzahnung (v.10, 11)
- 54: Seitenfläche (v.10, 11)
- 55: Seitenfläche (v.10, 11)
- 56: Führungsseite (v.10, 11)
- 57: Führungsseite (v.10, 11)
- 58: Grundwand (v. 40, 41)
- 59: Steg (v. 40, 41)
- 60: Steg (v.40, 41)
- 61: Führungsfläche (v.40, 41)
- 62: Führungsfläche (v.40, 41)
- 63: Proximales Ende (v.39, 3)
- 64: Breite (v.34, 39)
- 65: Öffnung (v.34)
- 66: Mittellinie (v.65)
- 67: Mittellinie (v.68)
- 68: Gewindebohrung (v.29)
- 69: Antriebswelle (v.33)
- 70: Verbindungselement (v.33)
- 71: Wellenende I (v.69)
- 72: Wellenende II (v.69)
- 73: Funktionsbereich I (v.69)
- 74: Funktionsbereich II (v.69)
- 75: Funktionsbereich III (v.69)
- 76: Feingewinde (v.69)
- 77: Flansch I (v.69)
- 78: Flansch II (v.69)
- 79: Zylindrischer Zapfen
- 80: Öffnung (v. 35)
- 81: Reinigungsöffnung (v.68)
- 82: Distales Ende (v.29)
- 83: Proximales Ende (v.29)
- 84: Außenwand (v.40, 41)
- 85: Wanddicke (v.40, 41)
- 86: Breite (v.38)
- 87: Abstand (v.10, 11)
- 88: Höhe (v. 59, 60)
- 89: Freies Ende (v.44)
- 90: Verdrillung (v.44)

- 91: Abstand (v.40, 41)
- 92: Breite (v.10, 11 )

## Patentansprüche

1. Medizinisches Instrument (1), zum Okkludieren von schlauchförmigen organischen Körperteilen, welches aus zwei, beweglich miteinander scherenartig verbundenen Schenkeln (5, 6) besteht, die am distalen Ende (7) eine Greifeinrichtung (9) aufweisen, die zwei Greifelemente (10, 11) umfasst und am proximalen Ende (8) aus einer Betätigungseinrichtung (4) besteht, die zwei Griffelemente (12, 13) umfasst, sowie nahe der Griffelemente (12, 13) aus einer Feststellvorrichtung (14) besteht, die Rastzähne (17) aufweisende Klinken (15, 16) am Schenkel (5, 6) umfasst, wodurch die Greifelemente (10, 11) in einer von mehreren Stellungen eingestellt werden können, wobei das zerleg- und komplettierbare chirurgische Instrument (1) aus einer Bedieneinrichtung (2), einer stufenlos fassenden und klemmenden Gefäßklemme (3) die ein Körperelement (39), ein Arbeitsmittel (30), eine Verstelleinrichtung (33), und eine Koppeleinrichtung (38) umfasst und einem Betätigungswerkzeug (25) gebildet wird,
wobei
die am distalen Ende (7) der Greifeinrichtung (9) ausgebildeten Greifelemente (10, 11) länglich sind und eine u-förmige und im Querschnitt trapezförmige Ausgestaltung aufweisen und mit einem Betrag, der sich aus der Breite (86) des Körperelementes (39) der Gefäßklemme (3) ergibt, parallel beabstandet sind und die innen liegenden Arbeitsflächen (23, 24) der Greifelemente (10, 11) eine strukturierte Oberfläche aufweisen und ein Schenkel (5) und eine Klinke (15) der Bedieneinrichtung (2) Träger eines daran angeordneten Führungselementes (18) ist, welches aus einem Hohlzylinder (19) gebildet wird und dessen Länge (20) der Gesamtlänge eines Schenkels (5) einschließlich Griffelement (12) entspricht, wobei der Hohlzylinder (19) aus einem metallischen Rohr besteht, welches mit Hilfe eines thermischen Verfahrens, an dem Schenkel (5) befestigt ist und eine durchgehende Bohrung (21) aufweist, die der Aufnahme, Führung und Entnahme des Betätigungswerkzeuges (25) dient, welches zur Betätigung der Verstelleinrichtung (33) eingesetzt wird, und wobei das Betätigungswerkzeug (25)
aus einer zylindrischen Führungstange (26) besteht, die länger ist als die Länge (20) des Hohlzylinders (19) und die der korrespondierenden Bohrung (21) des Hohlzylinders (19) spielfrei angepasst ist und an einem Ende ein Handgriff (28) mit einer Drehrichtungsmarkierung und am anderen Ende eine Klinge (27) aufweist, wobei die Größe der Klinge (27) kleiner als die Bohrung (21) im Hohlzylinder (19) ist, und wobei die
stufenlos fassende- und klemmende Gefäßklemme (3)
ein Körperelement (39), ein Zug-und Druckelement (29), eine Koppeleinrichtung (38) und eine Verstelleinrichtung (33) in Kombination mit einer Arretierungseinrichtung (43) umfasst, wobei die Koppeleinrichtung (38) aus zwei Haltebacken (40, 41) und die Verstelleinrichtung (33) aus einer abgewinkelten Halterung (34), einer Antriebswelle (69) und einem Verstellelement (35) und die Arretierungseinrichtung (43) aus einer Rastfeder (44) und dem Verstellelement (35), gebildet wird.

2. Medizinisches Instrument (1), nach Anspruch 1, **dadurch gekennzeichnet, dass** die abgewinkelte Halterung (34) die gleiche Breite (64) des Körperelementes (39) aufweist und am proximalen Ende (63) senkrecht zur Längsachse des Körperelementes (39) und senkrecht zum Zug- und Druckelement (29) angeordnet ist und einen quaderförmigen Körper bildet, der eine Bohrung (65) zur Aufnahme und Führung eines Verbindungselementes (70) bestehend aus einer Antriebswelle (69), die mit einem Wellenende (71) in das verschiebbare Zug- und Druckelement (29) eingreift und mit dem anderen Wellenende (72) das Verstellelement (35) aufnimmt.

3. Medizinisches Instrument (1), nach Anspruch 2, **dadurch gekennzeichnet, dass** die
Antriebswelle (69) das Verbindungselement (70) zwischen dem Zug-und Druckelement (29) und dem Verstellelement (35) darstellt und drei Funktionsbereiche I bis III (73, 74, 75) aufweist, wobei der eine Funktionsbereich I (73) dem Wellenende I (71) entspricht, welches als Gewindebolzen mit Feingewinde (76) ausgebildet ist, der zweite Funktionsbereich II (74) im mittleren Teil der Antriebswelle (69) aus zwei beabstandeten Flanschen (77, 78) besteht und der dritte Funktionsbereich III (75) dem Wellenende II (72) entspricht, welches als zylindrischer Zapfen (79) ausgebildet ist.

4. Medizinisches Instrument (1), nach Anspruch 1, **dadurch gekennzeichnet, dass** am Körperelement (39) auf der abgewandten Seite der Halterung (34) eine Rastfeder (44) angeordnet ist, die mit dem Verstellelement (35) eine Arretierungseinrichtung (43) bildet, wobei die Rastfeder (44) aus Federstahl besteht und zwei Schenkel (46, 47) aufweist, die rechtwinklig zueinander angeordnet sind.

5. Medizinisches Instrument nach Anspruch 1, **dadurch gekennzeichnet, dass** das Verstellelement (35) aus einem Schraubenkopfteil (36) und einem Schraubenbolzenteil (45) gebildet wird, wobei der Schraubenkopfteil (36) am Umfang ein Rastprofil (37) und ein innen liegendes Formelement (50) zur formschlüssigen Verbindung mit der Klinge (27) des Betätigungswerkzeuges (25) aufweist und dass der Schraubenbolzenteil (45) ein Feingewinde und einen Flansch (77) aufweist.

6. Medizinisches Instrument nach einem der vorhergehenden Ansprüche, **dadurch gekennzeichnet, dass** die Koppeleinrichtung (38) aus zwei Haltebacken (40, 41) gebildet wird, die einstückig mit dem Körperelement (39) verbunden und am proximalen Ende (63) der Gefäßklemme (3) und seitlich in Längsrichtung am Körperelement (39) angeordnet sind, wobei die Haltebacken (40, 41) durch die Breite (64) des Körperelementes (39) parallel beabstandet sind.

7. Medizinisches Instrument nach Anspruch 6, **dadurch gekennzeichnet, dass** jede Haltebacke (40, 41) im Querschnitt u-förmig ausgebildet ist und eine Grundwand (58) und zwei parallel beabstandete Stege (59, 60) umfasst, wobei die Außenwand (84) der Grundwand (58) die dem Körperelement (39) zugewandte Seite ist und die innen liegende Fläche die Anlagefläche (48, 49) für die Greifelemente (10, 11) bildet.

8. Medizinisches Instrument nach Anspruch 7, **dadurch gekennzeichnet, dass** die Stege (59, 60) innen liegende Führungsflächen (61, 62) aufweisen, die einen Abstand (91) bilden, der mit dem Abstand der Führungsseiten (56, 57), der durch die Breite (92) der Greifklemmen (40, 41) vorgegeben ist, korrespondiert.

9. Medizinisches Instrument nach Anspruch 8, **dadurch gekennzeichnet, dass** die innen liegenden Führungsflächen (61, 62) der Stege (59, 60) eine verjüngende Neigung zur Anlagefläche (48, 49) aufweisen, wobei die Neigung der Führungsflächen (61, 62) mit der verjüngenden Neigung der Seitenflächen (54, 55) die in Richtung der Arbeitsfläche (23, 24) der Greifelemente (10, 11) verlaufen, korrespondiert.

10. Medizinisches Instrument (1), nach einem der vorhergehenden Ansprüche, **dadurch gekennzeichnet, dass** die Klinge (27) des Betätigungswerkzeuges (25) aus einem außen Inbusprofil oder einem außen Kreuzschlitzprofil oder einem außen Torxprofil gebildet ist.

## Claims

1. A medical instrument (1) for the occluding of tube-shaped organic body parts, which consist of two shanks (5, 6) connected movably with each other in a scissor-like manner, which have at the distal end (7) a gripping arrangement (9), which comprises two gripping elements (10, 11) and at the proximal end (8) consists of an actuating arrangement (4), which comprises two gripping elements (12, 13), and close to the gripping elements (12, 13) consists of a locking device (14), which comprises ratchets (15, 16), having detent teeth (17), on the shank (5, 6), whereby the gripping elements (10, 11) can be set in one of several positions, wherein the surgical instrument (1), which is able to be disassembled and completed, is formed from an operating arrangement (2), a continuously grasping and clamping vascular clamp (3) which comprises a body element (39), a working means (30), an adjustment arrangement (33) and a coupling arrangement (38), and from an actuating tool (25),
wherein
the gripping elements (10, 11) constructed at the distal end (7) of the gripping arrangement (9) are elongate and have a U-shaped configuration which is trapezoidal in cross-section, and are spaced apart in parallel by an amount which is produced from the width (86) of the body element (39) of the vascular clamp (3), and the working surfaces (23, 24) of the gripping elements (10, 11) lying on the inside have a structured surface, and a shank (5) and a ratchet (15) of the operating arrangement (2) is a carrier of a guide element (18) arranged thereon, which is formed from a hollow cylinder (19), and the length (20) thereof corresponds to the overall length of a shank (5) including the gripping element (12), wherein the hollow cylinder (19) consists of a metallic tube which is fastened by means of a thermal process on the shank (5) and has a continuous bore (21) which serves for the receiving, guiding and removal of the actuating tool (25), which is used for actuation of the adjustment arrangement (33), and wherein
the actuating tool (25)
consists of a cylindrical guide rod (26), which is longer than the length (20) of the hollow cylinder (19) and which is adapted free of play to the corresponding bore (21) of the hollow cylinder (19) and has at one end a handle (28) with a rotary direction marking and at the other end a blade (27), wherein the size of the blade (27) is smaller than the bore (21) in the hollow cylinder (19), and wherein the continuously grasping and clamping vascular clamp (3) comprises a body element (39), a push-pull element (29), a coupling arrangement (38) and an adjustment arrangement (33) in combination with an arresting arrangement (43), wherein the coupling arrangement (38) is formed from two retaining jaws (40, 41) and the adjustment arrangement (33) is formed from an angled holder (34), a drive shaft (69) and an adjustment element (35), and the arresting arrangement (43) is formed from a detent spring (44) and from the adjustment element (35).

2. The medical instrument (1) according to Claim 1, **characterized in that** the angled holder (34) has the same width (64) as the body element (39) and is arranged at the proximal end (63) perpendicularly to the longitudinal axis of the body element (39) and
perpendicularly to the push-pull element (29) and forms a cuboid body having a bore (65) to receive and guide a connecting element (70) consisting of a drive shaft (69), which engages with a shaft end (71) into the displaceable push-pull element (29) and receives by the other shaft end (72) the adjustment element (35).

3. The medical instrument (1) according to Claim 2, **characterized in that** the drive shaft (69) constitutes the connecting element (70) between the push-pull element (29) and the adjustment element (35) and has three functional zones I to III (73, 74, 75), wherein the one functional zone I (73) corresponds to the shaft end (71), which is constructed as a threaded bolt with a fine thread (76), the second functional zone II (74) in the middle part of the drive shaft (69) consists of two spaced-apart flanges (77, 78), and the third functional zone III (75) corresponds to the shaft end II (72), which is constructed as a cylindrical pin (79).

4. The medical instrument (1) according to Claim 1, **characterized in that** on the body element (39) on the reverse side of the holder (34) a detent spring (44) is arranged, which forms an arresting arrangement (43) with the adjustment element (35), wherein the detent spring (44) consists of spring steel and has two shanks (46, 47), which are arranged at right-angles to each other.

5. The medical instrument according to Claim 1, **characterized in that** the adjustment element (35) is formed from a screw head part (36) and a screw bolt part (45), wherein the screw head part (36) has on the circumference a detent profile (37) and a shaped element (50) lying on the inside for the form-fitting connection with the blade (27) of the actuating tool (25), and that the screw bolt part (45) has a fine thread and a flange (77).

6. The medical instrument according to one of the preceding claims, **characterized in that** the coupling arrangement (38) is formed from two retaining jaws (40, 41), which are connected in one piece with the body element (39) and are arranged at the proximal end (63) of the vascular clamp (3) and laterally in longitudinal direction on the body element (39), wherein the retaining jaws (40, 41) are spaced apart in parallel by the width (64) of the body element (39).

7. The medical instrument according to Claim 6, **characterized in that** each retaining jaw (40, 41) is constructed so as to be U-shaped in cross-section and comprises a base wall (58) and two crosspieces (59, 60) spaced apart in parallel, wherein the outer wall (84) of the base wall (58) is the side facing the body element (39) and the surface lying on the inside forms the contact surface (48, 49) for the gripping elements (10, 11).

8. The medical instrument according to Claim 7, **characterized in that** the crosspieces (59, 60) have guide surfaces (61, 62) lying on the inside, which form a distance (91) which corresponds to the distance of the guiding sides (56, 57) which is provided by the width (92) of the gripping clamps (40, 41).

9. The medical instrument according to Claim 8, **characterized in that** the guide surfaces (61, 62) of the crosspieces (59, 60) lying on the inside have a tapering inclination to the contact surface (48, 49), wherein the inclination of the guide surfaces (61, 62) corresponds to the tapering inclination of the side surfaces (54, 55) which run in the direction of the working surface (23, 24) of the gripping elements (10, 11).

10. The medical instrument (1) according to one of the preceding claims, **characterized in that** the blade (27) of the actuating tool (25) is formed from an external hexagon profile or an external cross slot profile or an external torx profile.

## Revendications

1. Instrument médical (1) permettant d'obturer des parties organiques corporelles de forme tubulaire, qui consiste en deux branches (5, 6) reliées entre elles de manière mobile à la manière de ciseaux et présentant à leur extrémité distale (7) un dispositif préhenseur (9) qui comprend deux éléments préhenseurs (10, 11) et consiste à son extrémité proximale (8) en un mécanisme d'actionnement (4) qui comprend deux éléments préhenseurs (12, 13), ainsi que près des éléments préhenseurs (12, 13) en un dispositif d'arrêt (14) qui comprend des lames (15, 16) présentant des dents d'enclenchement (17) sur la branche (5, 6), grâce auxquelles les éléments préhenseurs (10, 11) peuvent être réglés à une ou plusieurs positions, l'instrument chirurgical pouvant être démonté et complété (1) étant constitué d'un dispositif de manoeuvre (2), d'une pince vasculaire (3) saisissant et bloquant progressivement, qui comprend un élément corporel (39), un moyen opératoire (30), un dispositif de réglage (33) et un dispositif de couplage (38), et en un outil d'actionnement (25),
les éléments préhenseurs (10, 11) réalisés à l'extrémité distale (7) du dispositif préhenseur (9) étant allongés et présentant une conformation en forme de U et trapézoïdale en section transversale et étant espacés parallèlement dans une mesure résultant de la largeur (86) de l'élément corporel (39) de la pince vasculaire (3) et les surfaces opératoires (23, 24) situées à l'intérieur des éléments préhenseurs (10, 11) présentant une surface structurée et une branche, (5) et une lame (15) du dispositif de manoeuvre (2) étant porteuse d'un élément de guidage (18) disposé dessus et constitué d'un cylindre creux (19) et sa longueur (20) correspondant à la longueur totale d'une branche (5), y compris l'élément préhenseur (12), le cylindre creux (19) étant composé d'un tube métallique qui est fixé à l'aide d'un procédé thermique sur la branche (5) et présente un alésage traversant (21) permettant de recevoir, guider et enlever l'outil d'actionnement (25) qui est utilisé pour actionner le dispositif de réglage (33),
l'outil d'actionnement (25) étant composé d'une barre de guidage cylindrique (26) qui est plus longue que la longueur (20) du cylindre creux (19) et qui est adaptée sans jeu à l'alésage correspondant (21) du cylindre creux (19) et présente à une extrémité une poignée (28) portant un marquage de sens de rotation et à l' autre extrémité une lame (27), la taille de la lame (27) étant plus inférieure à celle de l'alésage (21) du cylindre creux (19), et
la pince vasculaire (3) saisissant et bloquant progressivement comprenant un élément corporel (39), un élément de traction et de pression (29), un système de couplage (38) et un système de réglage (33) combiné à un système d'arrêt (43), le système de couplage (38) étant constitué de deux mâchoires de retenue (40, 41) et le système de réglage (33) étant composé d'un support coudé (34), d'un arbre moteur (69) et d'un élément de réglage (35) et le système d'arrêt (43) d'un ressort d'enclenchement (44) et de l'élément de réglage (35).

2. Instrument médical (1) selon la revendication 1, **caractérisé en ce que** le support coudé (34) a la même largeur (64) que l'élément corporel (39) et est disposé à l'extrémité proximale (63) perpendiculairement à l'axe longitudinal de l'élément corporel (39) et perpendiculairement à l'élément de traction et de pression (29) et forme un corps quadrangulaire qui reçoit un alésage (65) destiné à recevoir et à guider un élément de liaison (70) composé d'un arbre moteur (69) qui s'engrène par un bout d'arbre (71) dans l'élément de traction et de pression déplaçable (29) et reçoit par son autre bout d'arbre (72) l'élément de réglage (35).

3. Instrument médical (1) selon la revendication 1, **caractérisé en ce que** l'arbre moteur (69) constitue l'élément de liaison (70) entre l'élément de traction et de pression (29) et l'élément de réglage (35) et présente trois plages fonctionnelles I à III (73, 74, 75), la plage fonctionnelle I (73) correspondant au bout d'arbre I (71) qui est réalisé sous forme de goujon fileté à filetage fin (76), le deuxième bout d'arbre II (74) étant composé dans la partie centrale de l'arbre moteur (69) de deux brides espacées (77, 78) et la troisième plage fonctionnelle III (75) correspondant au bout d'arbre II (72) qui est réalisé sous forme de tourillon cylindrique (79).

4. Instrument médical (1) selon la revendication 1, **caractérisé en ce que**, sur l'élément corporel (39), du côté détourné du support (34), est disposé un ressort d'enclenchement (44) qui constitue avec l'élément de réglage (35) un système d'arrêt (43), le ressort d'enclenchement (44) étant composé d'acier à ressort et présentant deux branches (46, 47) qui sont disposées orthogonalement l'une par rapport à l'autre.

5. Instrument médical selon la revendication 1, **caractérisé en ce que** l'élément de réglage (35) est constitué d'un élément à tête de vis (36) et d'un élément à tête de boulon (45), l'élément à tête de vis (36) présentant sur sa circonférence un profil d'enclenchement (37) et un élément moulé situé à l'intérieur (50) pour la liaison en correspondance géométrique à la lame (27) de l'outil d'actionnement (25) et **en ce que** l'élément à tête de boulon (45) présente un filetage fin et une bride (77).

6. Instrument médical selon une des revendications précédentes, **caractérisé en ce que** le système de couplage (38) est constitué de deux mâchoires de retenue (40, 41) qui sont reliées en un bloc à l'élément corporel (39) et sont disposées à l'extrémité proximale (63) de la pince vasculaire (3) et latéralement dans le sens longitudinal au niveau de l'élément corporel (39), les mâchoires de retenue (40, 41) étant espacées parallèlement par la largeur (64) de l'élément corporel (39).

7. Instrument médical selon la revendication 6, **caractérisé en ce que** chaque mâchoire de retenue (40, 41) est réalisée en forme de U dans sa section transversale et comprend une paroi de base (58) et deux traverses espacées (59, 60), la paroi extérieure (84) de la paroi de base (58) étant le côté tourné vers l'élément corporel (39) et la surface située à l'intérieur constituant la surface d'appui (48, 49) pour les éléments préhenseurs (10, 11).

8. Instrument médical selon la revendication 7, **caractérisé en ce que** les traverses (59, 60) présentent des surfaces de guidage situées à l'intérieur (61, 62) qui forment un écart (91) qui correspond à l'écart entre les côtés de guidage (56, 57) qui est prescrit par la largeur (92) des pinces de préhension (40, 41).

9. Instrument médical selon la revendication 8, **caractérisé en ce que** les surfaces de guidage situées à l'intérieur (61, 62) des traverses (59, 60) présentent une tendance à se rétrécir vers la surface de contact (48, 49), cette tendance des surfaces de guidage (61, 62) correspondant à la tendance à se rétrécir des surfaces latérales (54, 55) qui s'étendent en direction de la surface opératoire (23, 24) des éléments préhenseurs (10, 11).

10. Instrument médical (1) selon une des revendications précédentes, **caractérisé en ce que** la lame (27) de l'outil d'actionnement (25) est constituée d'un profil à pans creux extérieur ou d'un profil cruciforme extérieur ou d'un profil torx extérieur.
